# EUROPEAN PATENT APPLICATION

(11) **EP 4 691 266 A1**
(43) Date of publication of application: **11.02.2026**
(21) Application number: 24780198.8
(22) Date of filing: 25.03.2024
(51) Int. Cl.: A23L 33/105, A61K 36/185, A61P 21/00, A61P 25/00, A61P 25/16, A61P 25/28

(54) **AGENT AND METHOD FOR MAINTAINING OR IMPROVING CEREBRAL FUNCTION USING CRUDE DRUG**

(30) Priority: 24.03.2023 JP 2023048891
(71) Applicant: Cerebro Pharma Inc., Osaka-shi, Osaka 541-0058 (JP)
(72) Inventor: TOMIYAMA, Takami, Osaka-shi, Osaka 541-0058 (JP); UMEDA, Tomohiro, Osaka-shi, Osaka 541-0058 (JP); MATSUDA, Kazunori, Osaka-shi, Osaka 541-0058 (JP)
(74) Representative: HGF
(86) International application number: PCT/JP2024/011731
(87) International publication number: WO 2024/204081

(57) **Abstract**

A brain function maintenance or improvement agent is provided that contains herbal medicine selected from ma̅maki leaves, fruit, and seeds.

## Description

### TECHNICAL FIELD

The present invention relates to an agent and a method using an herbal medicine for maintaining or improving cerebral function, promoting nerve cell repair or inducing neurogenesis, removing a neurodegenerative disease-causing protein that accumulates in the brain, or treating or preventing a neurodegenerative disease, as well as food and pharmaceutical products containing such an agent.

### BACKGROUND ART

With the aging of society and the westernization of lifestyles and eating habits, the number of dementia patients is rapidly increasing worldwide. The enormous social costs for medical and nursing care and the economic losses due to the decline in the labor force of patients and their families have become a major social issue.

Representative forms of dementia include Alzheimer's disease, frontotemporal dementia, and Lewy body dementia. These are all neurodegenerative diseases, and dementia caused by them is called degenerative dementia. In these diseases, specific proteins are thought to aggregate and accumulate in the nervous system, leading to the death of nerve cells and the onset of dementia. Specifically, Aβ and tau accumulate in the brain in Alzheimer's disease, tau and TDP-43 in frontotemporal dementia, and α-synuclein in Lewy body dementia (Non-Patent Literature 1: Spires-Jones et al., Acta Neuropathol., (2017), 134[2]:187-205).

As anti-dementia drugs, development is underway for drugs that suppress the production of these causative proteins or remove them from the brain. Candidates for such anti-dementia drugs include Aβ-producing enzyme inhibitors (β-secretase and γ-secretase) for Alzheimer's disease (Non-Patent Literature 2: Luo et al., Cell & Biosci., (2022), 12:2), Aβ vaccines (Non-Patent Literature 3: Valiukas et al., Vaccines, (2022), 10[9]:1527) and Aβ antibodies (Non-Patent Literature 4: Song et al., Transl. Neurodegener., (2022), 11:18); tau vaccines (Non-Patent Literature 5: Medina, Int. J. Mol. Sci., (2018), 19[4]:1160) and tau antibodies for frontotemporal dementia (Non-Patent Literature 6: Ji et al., Drugs, (2021), 81[10]:1135-1152); and α-synuclein vaccines and α-synuclein antibodies for Lewy body dementia (Non-Patent Literature 7: Alzforum website, FBRI LLC, search results for alpha-synuclein targets, https://www.alzforum.org/therapeutics/search?fda_statuses=&target_types%5B%5D=3341 6&therapy_types=&conditions=&keywords-entry=&keywords=, according to search conducted in December 2022).

However, most of the anti-dementia drug candidates developed so far have failed to demonstrate the expected efficacy in clinical trials with dementia patients (Non-Patent Literature 8: Asher et al., Life Sciences, (2022), 306:120861).

The leaves of the Hawaiian native herb ma̅maki (*Pipturus albidus*) are used as a popular herbal tea ingredient in Hawaii. In Hawaii, ma̅maki, particularly its fruit, has a history of being used as a natural folk remedy to regulate blood sugar, blood pressure, and cholesterol levels, relieve stress and fatigue, and reduce inflammation (Non-Patent Literature 9: Rafii et al., Alzheimers Dement., (2022), 1-7). However, its effect on dementia is not known.

### LIST OF CITATIONS

### Non-Patent Literature

[Non-Patent Literature 1] Spires-Jones et al., Acta Neuropathol., (2017), 134[2]:187-205
[Non-Patent Literature 2] Luo et al., Cell & Biosci., (2022), 12:2
[Non-Patent Literature 3] Valiukas et al., Vaccines, (2022), 10[9]:1527
[Non-Patent Literature 4] Song et al., Transl. Neurodegener., (2022), 11:18
[Non-Patent Literature 5] Medina, Int. J. Mol. Sci., (2018), 19[4]:1160
[Non-Patent Literature 6] Ji et al., Drugs, (2021), 81[10]:1135-1152
[Non-Patent Literature 7] Alzforum website, FBRI LLC, search results for alpha-synuclein targets, https://www.alzforum.org/therapeutics/search?fda_statuses=&target_types%5B%5D=3341 6&therapy_types=&conditions=&keywords-entry=&keywords=, according to search conducted in December 2022
[Non-Patent Literature 8] Asher et al., Life Sciences, (2022), 306:120861
[Non-Patent Literature 9] Rafii et al., Alzheimers Dement., (2022), 1-7
[Non-Patent Literature 10] Chun et al., Native Hawaiian medicines, First People's Productions Honolulu, (1994), 216-217
[Non-Patent Literature 11] Afzal et al., Molecules, (2022), 27[21]:7604
[Non-Patent Literature 12] Payne et al., Biomolecules, (2022), 12[3]:371
[Non-Patent Literature 13] Li et al., Neuroscience, (2009), 159[4]:1208-15
[Non-Patent Literature 14] Yoo et al., Phytother Res., (2010), 24[7]:1065-70
[Non-Patent Literature 15] Colucci-D'Amato et al., Int. J. Mol. Sci., (2020), 21[20]:7777
[Non-Patent Literature 16] Horgusluoglu et al., Am. J. Med. Gene. B. Neuropsychiatr. Genet., (2017), 174[1]:93-112
[Non-Patent Literature 17] Socala et al., Int. J. Mol. Sci., (2020), 22[1]:107
[Non-Patent Literature 18] Gao et al., Drug Des. Devel. Ther., (2020), 14:1705-1716
[Non-Patent Literature 19] Singh et al., Oxid. Med. Cell Longev., (2020), 2020:6571484
[Non-Patent Literature 20] Liu et al., Drug Des. Devel. Ther., (2020), 14:51-60
[Non-Patent Literature 21] Tahir et al., Biomed. Pharmacother., (2021), 137:111253
[Non-Patent Literature 22] Xu et al., Behav. Brain Res., (2014), 264:173-80
[Non-Patent Literature 23] Sun et al., J. Neuroinflammation, (2021), 18[1]:131
[Non-Patent Literature 24] Moghbelinejad et al., Toxicol Lett., (2014), 224[1]:108-13
[Non-Patent Literature 25] Umeda et al., Am. J. Pathol., (2013), 183[1]:211-25
[Non-Patent Literature 26] Umeda et al., Ann. Clin. Transl. Neurol., (2015), 2[3]:241-55
[Non-Patent Literature 27] Sturchler-Pierrat et al., Proc. Natl. Acad. Sci. U.S.A., (1997), 94[24]:13287-92
[Non-Patent Literature 28] Van Dam et al., Eur. J. Neurosci., (2003), 17[2]:388-96
[Non-Patent Literature 29] Umeda et al., Front. Neurosci., (2021), 15:763476
[Non-Patent Literature 30] Lee et al., Proc. Natl. Acad. Sci. U.S.A., (2002), 99[13]:8968-73
[Non-Patent Literature 31] Umeda et al., Int. J. Mol. Sci., (2021), 22:8453
[Non-Patent Literature 32] Liu et al., Neuron, (2016), 90[3]:521-534
[Non-Patent Literature 33] Hatanaka et al., Biomedicines, (2022), 10[5]:1080

### SUMMARY OF INVENTION

### PROBLEM TO BE SOLVED BY THE INVENTION

A problem to be solved by the present invention is to provide a novel means for maintaining or improving cerebral function, promoting nerve cell repair or inducing neurogenesis, removing a neurodegenerative disease-causing protein that accumulates in the brain, or treating or preventing a neurodegenerative disease.

### MEANS TO SOLVE THE PROBLEM

As a result of intensive research, the present inventors have found that an herbal medicine selected from ma̅maki leaves, fruit, and seeds, each in a specific form, has various effects such as maintaining or improving cerebral function, promoting nerve cell repair or inducing neurogenesis, removing a neurodegenerative disease-causing protein that accumulates in the brain, or treating or preventing a neurodegenerative disease, thereby completing the present invention.

Accordingly, aspects of the present invention include, for example, the following.

### [Aspect 1]

An agent for maintaining or improving a cerebral function, comprising an herbal medicine selected from ma̅maki leaves, fruit, and seeds.

### [Aspect 2]

The agent for maintaining or improving a cerebral function according to aspect 1, wherein the cerebral function is cognitive function.

### [Aspect 3]

An agent for promoting nerve cell repair or inducing neurogenesis, comprising an herbal medicine selected from ma̅maki leaves, fruit, and seeds.

### [Aspect 4]

An agent for removing a neurodegenerative disease-causing protein that accumulates in the brain, comprising an herbal medicine selected from ma̅maki leaves, fruit, and seeds.

### [Aspect 5]

The agent according to aspect 4, wherein the neurodegenerative disease-causing protein is one or more proteins selected from amyloid β (Aβ), tau, α-synuclein, TDP-43, FUS/TLS, polyglutamine, a protein resulting from RAN (repeat-associated non-ATG) translation, prion, and SOD-1.

### [Aspect 6]

An agent for treating or preventing a neurodegenerative disease, comprising an herbal medicine selected from ma̅maki leaves, fruit, and seeds.

### [Aspect 7]

The agent according to aspect 6, wherein the neurodegenerative disease is degenerative dementia.

### [Aspect 8]

The agent according to aspect 7, wherein the degenerative dementia is one or more dementias selected from Alzheimer's disease, frontotemporal dementia, Lewy body dementia, and dementia resulting from Parkinson's disease or amyotrophic lateral sclerosis.

### [Aspect 9]

The agent according to any one of aspects 1 to 8, wherein the herbal medicine selected from ma̅maki leaves, fruit, and seeds is a crushed product and/or an extract of ma̅maki leaves and/or fruit and/or seeds.

### [Aspect 10]

The agent according to any one of aspects 1 to 9, wherein the herbal medicine is administered to a subject at an amount of from 0.01 mg to 10 g per day.

### [Aspect 11]

A food product comprising the agent according to any one of aspects 1 to 10.

### [Aspect 12]

A pharmaceutical product comprising the agent according to any one of aspects 1 to 10.

### [Aspect 13]

A method for maintaining or improving a cerebral function in a subject, the method comprising administering to a subject in need thereof an herbal medicine selected from ma̅maki leaves, fruit, and seeds.

### [Aspect 14]

The method according to aspect 13, wherein the cerebral function is cognitive function.

### [Aspect 15]

A method for promoting nerve cell repair or inducing neurogenesis in a subject, the method comprising administering to a subject in need thereof an herbal medicine selected from ma̅maki leaves, fruit, and seeds.

### [Aspect 16]

A method for removing a neurodegenerative disease-causing protein that accumulates in the brain of a subject, the method comprising administering to a subject in need thereof an herbal medicine selected from ma̅maki leaves, fruit, and seeds.

### [Aspect 17]

The method according to aspect 16, wherein the neurodegenerative disease-causing protein is one or more proteins selected from amyloid β (Aβ), tau, α-synuclein, TDP-43, FUS/TLS, polyglutamine, a protein resulting from RAN (repeat-associated non-ATG) translation, prion, and SOD-1.

### [Aspect 18]

A method for treating or preventing a neurodegenerative disease in a subject, the method comprising administering to a subject in need thereof an herbal medicine selected from ma̅maki leaves, fruit, and seeds.

### [Aspect 19]

The method according to aspect 18, wherein the neurodegenerative disease is degenerative dementia.

### [Aspect 20]

The method according to aspect 19, wherein the degenerative dementia is one or more dementias selected from Alzheimer's disease, frontotemporal dementia, Lewy body dementia, and dementia resulting from Parkinson's disease or amyotrophic lateral sclerosis.

### [Aspect 21]

The method according to any one of aspects 13 to 20, wherein the herbal medicine selected from ma̅maki leaves, fruit, and seeds is a crushed product and/or an extract of ma̅maki leaves and/or fruit and/or seeds.

### [Aspect 22]

The method according to any one of aspects 13 to 21, wherein the herbal medicine is administered to a subject at an amount of from 0.01 mg to 10 g per day.

### EFFECT OF THE INVENTION

The present invention provides a novel means for maintaining or improving cerebral function, promoting nerve cell repair or inducing neurogenesis, removing a neurodegenerative disease-causing protein that accumulates in the brain, or treating or preventing a neurodegenerative disease by using an herbal medicine selected from ma̅maki leaves, fruit, and seeds, each in a specific form.

### BRIEF DESCRIPTION OF THE FIGURES

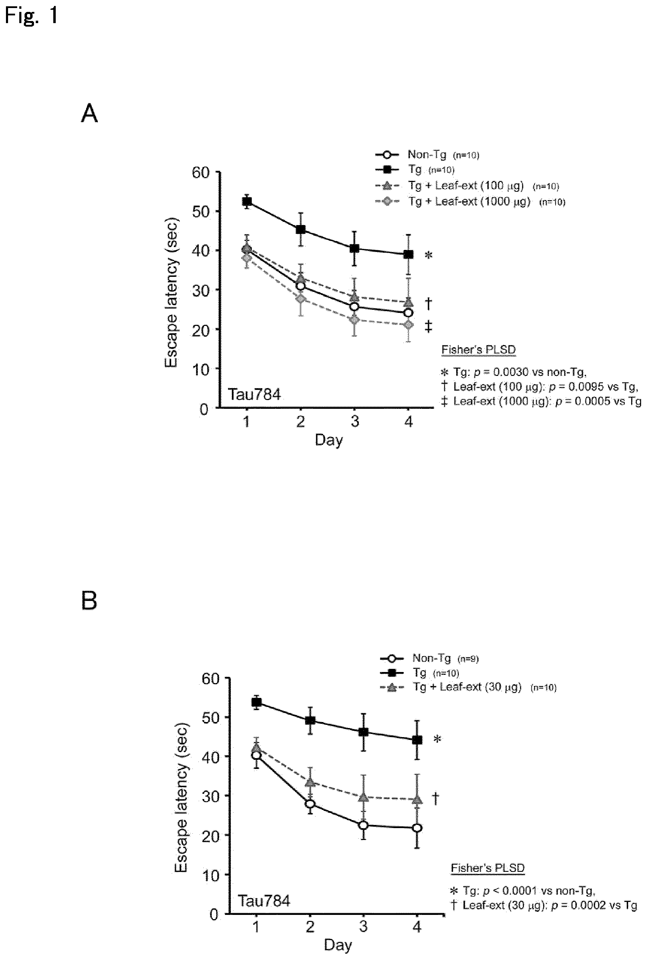

[Figure 1] Figure 1 is a graph showing the results of the Morris water maze test for Tau784 mice administered ma̅maki leaf hot water extract (Leaf-ext) orally for one month (labeled "Tg + Leaf-ext" in the table), in comparison with Tau784 mice administered water (labeled "Tg" in the table) and non-genetically modified mice (labeled "Non-Tg" in the table). Fig. 1A is a graph showing the results for the 1000 µg/day and 100 µg/day dose groups, and Fig. 1B is a graph showing the results for the 30 µg/day dose group.

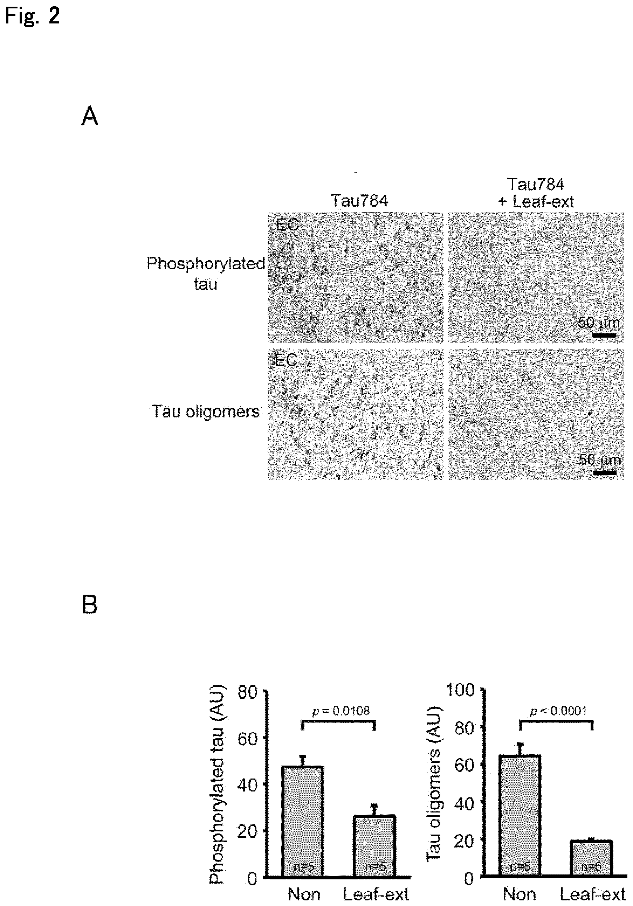

[Figure 2] Figure 2 shows tau pathology in the entorhinal cortex of Tau784 mice administered ma̅maki leaf hot water extract (Leaf-ext) at a dose of 100 µg/day orally for one month (labeled "Tau784 + Leaf-ext" in the table), in comparison with Tau784 mice administered water (labeled "Tau784" in the table). Fig. 2A shows photographs of the staining results for phosphorylated tau and tau oligomers, and Fig. 2B shows graphs of the quantitative values of the staining intensity in each photograph.

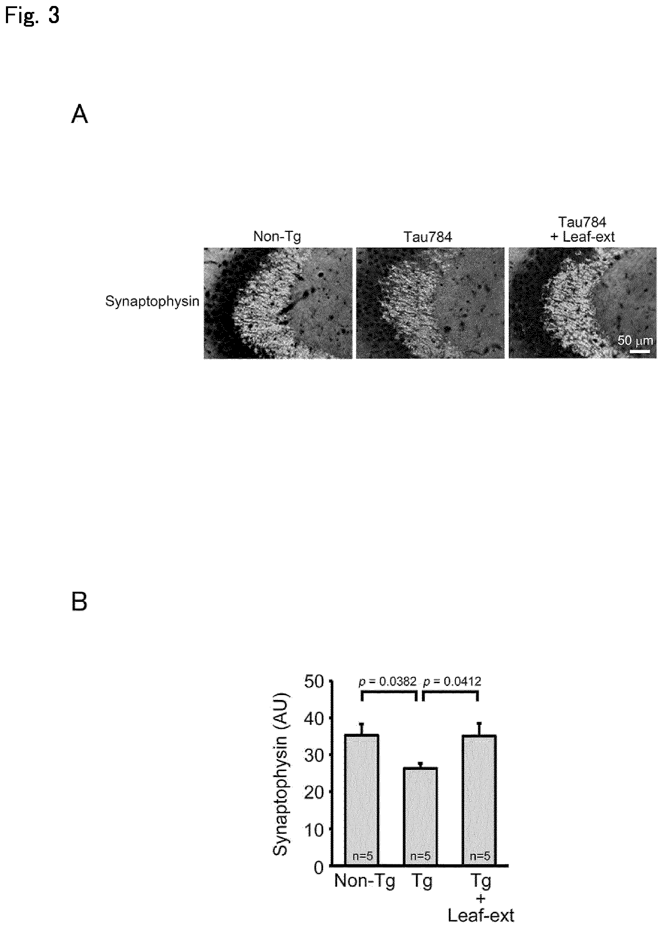

[Figure 3] Figure 3 shows synaptophysin pathology in the hippocampal CA2/3 region of Tau784 mice administered ma̅maki leaf hot water extract (Leaf-ext) at a dose of 100 µg/day orally for one month (labeled "Tau784 + Leaf-ext" in the table), in comparison with Tau784 mice administered water (labeled "Tau784" in the table) and non-genetically modified mice (labeled "Non-Tg" in the table). Fig. 3A shows photographs of the staining results for synaptophysin, and Fig. 3B shows graphs of the quantitative values of the staining intensity in each photograph.

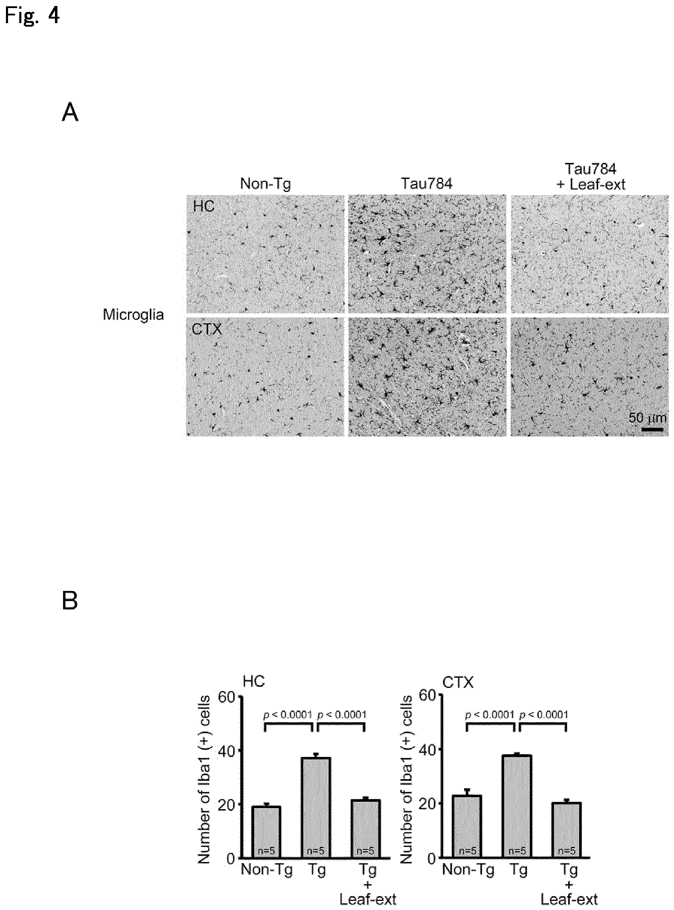

[Figure 4] Figure 4 shows microglia pathology in the hippocampus (HC) and cerebral cortex (CTX) of Tau784 mice administered ma̅maki leaf hot water extract (Leaf-ext) at a dose of 100 µg/day orally for one month (labeled "Tau784 + Leaf-ext" in the table), in comparison with Tau784 mice administered water (labeled "Tau784" in the table) and non-genetically modified mice (labeled "Non-Tg" in the table). Fig. 4A shows photographs of the staining results for activated microglia, and Fig. 4B shows graphs of the number of positive cells in each photograph.

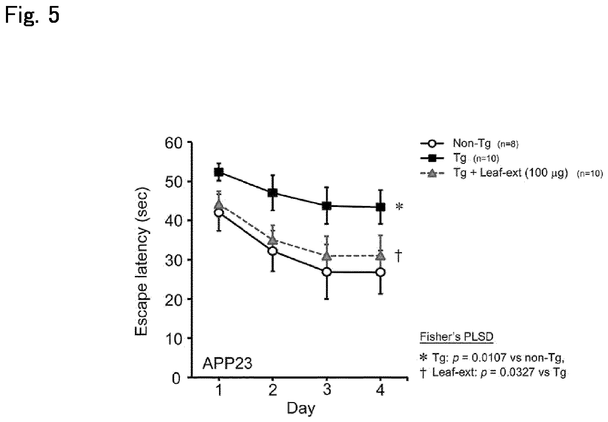

[Figure 5] Figure 5 is a graph showing the results of the Morris water maze test for APP23 mice administered ma̅maki leaf hot water extract (Leaf-ext) at a dose of 100 µg/day orally for one month (labeled "Tg + Leaf-ext" in the table), in comparison with APP23 mice administered water (labeled "Tg" in the table) and non-genetically modified mice (labeled "Non-Tg" in the table).

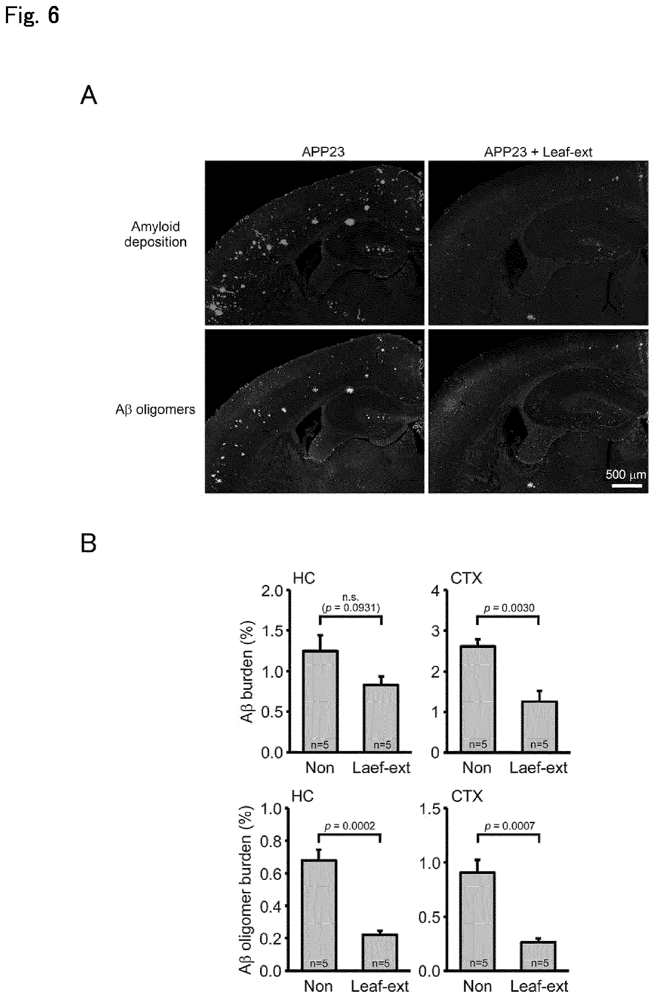

[Figure 6] Figure 6 shows amyloid pathology in the cerebral cortex and hippocampus of APP23 mice administered ma̅maki leaf hot water extract (Leaf-ext) at a dose of 100 µg/day orally for one month (labeled "APP23 + Leaf-ext" in the table), in comparison with APP23 mice administered water (labeled "APP23" in the table). Fig. 6A shows photographs of the staining results for amyloid deposition and Aβ oligomers, and Fig. 6B shows graphs of the quantitative values of the staining intensity in each photograph.

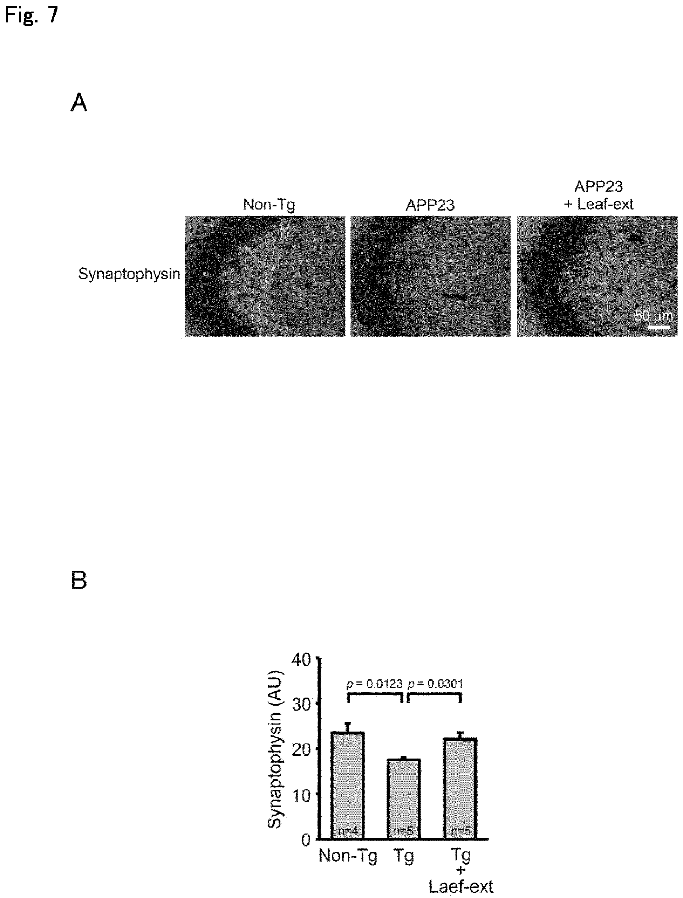

[Figure 7] Figure 7 shows synaptophysin pathology in the hippocampal CA2/3 region of APP23 mice administered ma̅maki leaf hot water extract (Leaf-ext) at a dose of 100 µg/day orally for one month (labeled "APP23 + Leaf-ext" in the table), in comparison with APP23 mice administered water (labeled "APP23" in the table) and non-genetically modified mice (labeled "Non-Tg" in the table). Fig. 7A shows photographs of the staining results for synaptophysin, and Fig. 7B shows graphs of the quantitative values of the staining intensity in each photograph.

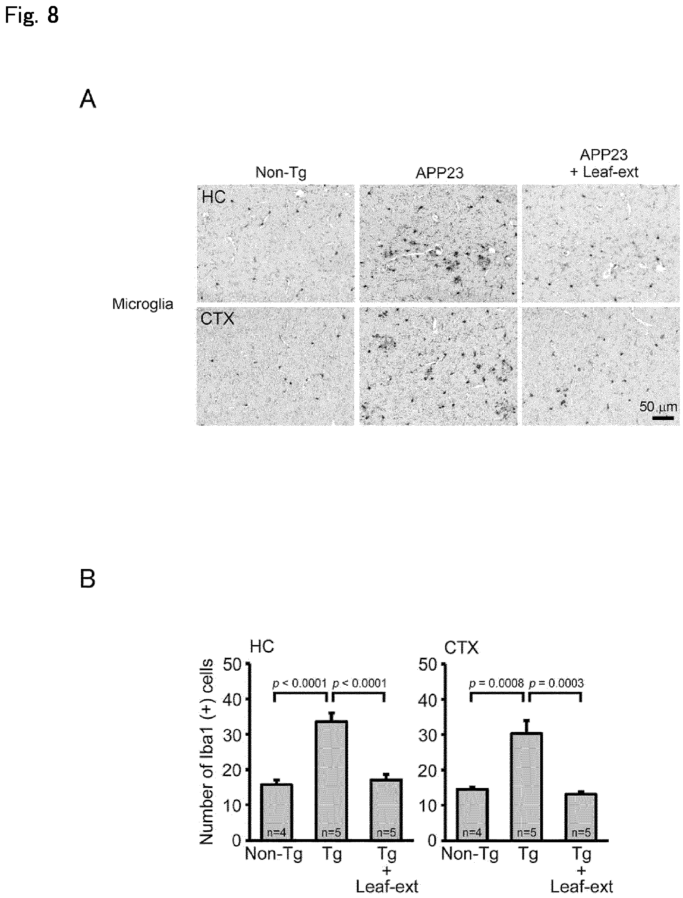

[Figure 8] Figure 8 shows microglia pathology in the hippocampus (HC) and cerebral cortex (CTX) of APP23 mice administered ma̅maki leaf hot water extract (Leaf-ext) at a dose of 100 µg/day orally for one month (labeled "APP23 + Leaf-ext" in the table), in comparison with APP23 mice administered water (labeled "APP23" in the table) and non-genetically modified mice (labeled "Non-Tg" in the table). Fig. 8A shows photographs of the staining results for activated microglia, and Fig. 8B shows graphs of the number of positive cells in each photograph.

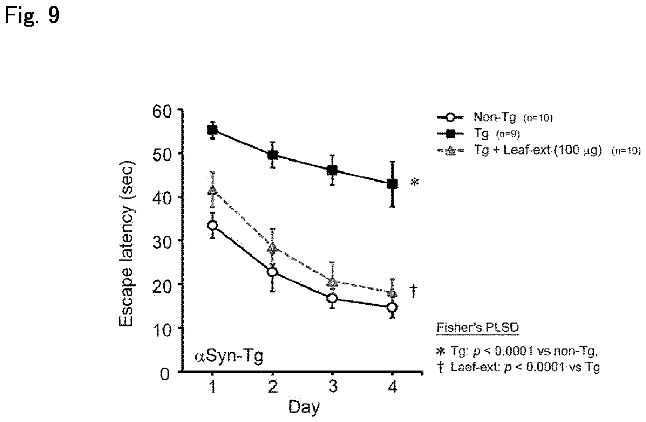

[Figure 9] Figure 9 is a graph showing the results of the Morris water maze test for Huα-Syn (A53T) mice administered ma̅maki leaf hot water extract (Leaf-ext) at a dose of 100 µg/day orally for one month (labeled "Tg + Leaf-ext" in the table), in comparison with Huα-Syn (A53T) mice administered water (labeled "Tg" in the table) and non-genetically modified mice (labeled "Non-Tg" in the table).

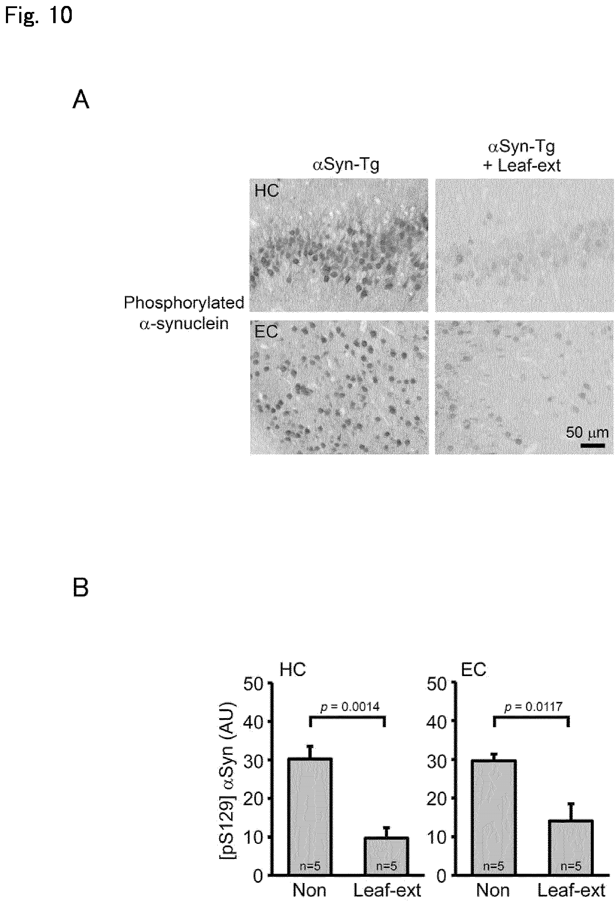

[Figure 10] Figure 10 shows α-synuclein pathology in the hippocampus (HC) and entorhinal cortex (EC) of Huα-Syn (A53T) mice administered ma̅maki leaf hot water extract (Leaf-ext) at a dose of 100 µg/day orally for one month (labeled "αSyn-Tg + Leaf-ext" in the table), in comparison with Huα-Syn (A53T) mice administered water (labeled "αSyn-Tg" in the table). Fig. 10A shows photographs of the staining results for phosphorylated α-synuclein, and Fig. 10B shows graphs of the quantitative values of the staining intensity in each photograph.

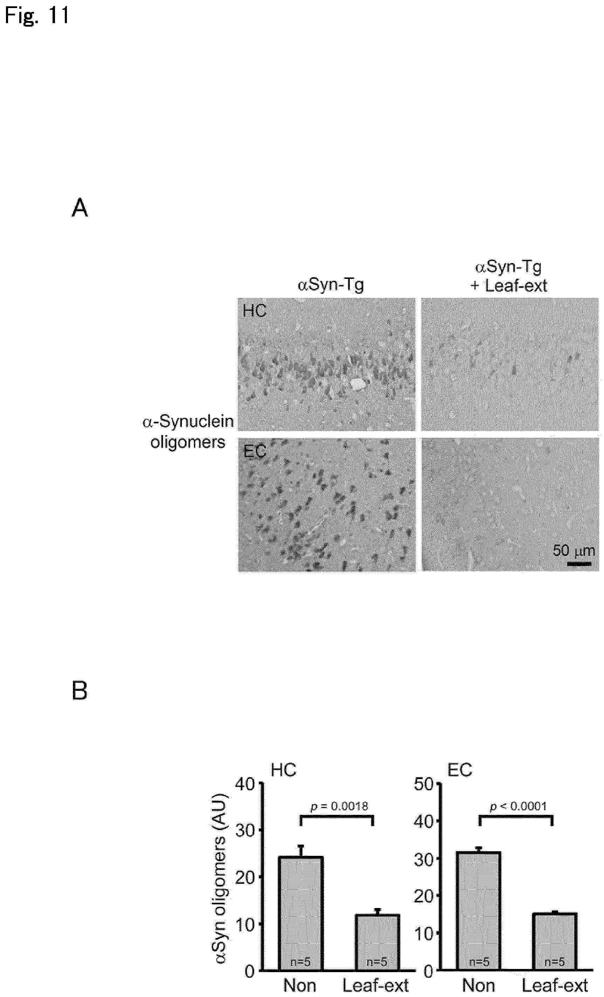

[Figure 11] Figure 11 shows α-synuclein pathology in the hippocampus (HC) and entorhinal cortex (EC) of Huα-Syn (A53T) mice administered ma̅maki leaf hot water extract (Leaf-ext) at a dose of 100 µg/day orally for one month (labeled "αSyn-Tg + Leaf-ext" in the table), in comparison with Huα-Syn (A53T) mice administered water (labeled "αSyn-Tg" in the table). Fig. 11A shows photographs of the staining results for α-synuclein oligomers, and Fig. 11B shows graphs of the quantitative values of the staining intensity in each photograph.

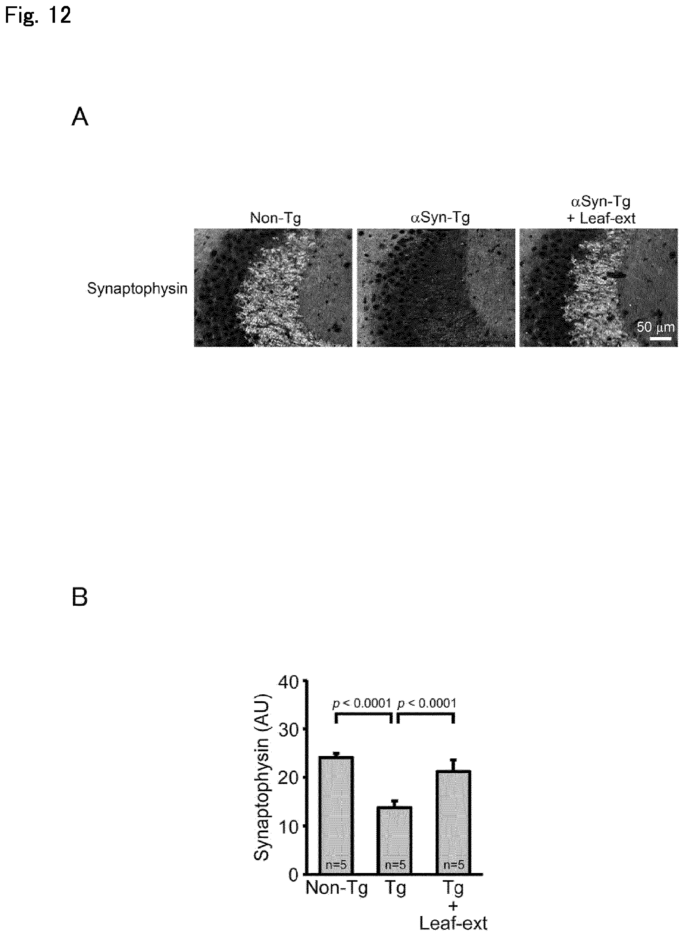

[Figure 12] Figure 12 shows synaptophysin pathology in the hippocampal CA2/3 region of Huα-Syn (A53T) mice administered ma̅maki leaf hot water extract (Leaf-ext) at a dose of 100 µg/day orally for one month (labeled "αSyn-Tg + Leaf-ext" in the table), in comparison with Huα-Syn (A53T) mice administered water (labeled "αSyn-Tg" in the table) and non-genetically modified mice (labeled "Non-Tg" in the table). Fig. 12A shows photographs of the staining results for synaptophysin, and Fig. 12B shows graphs of the quantitative values of the staining intensity in each photograph.

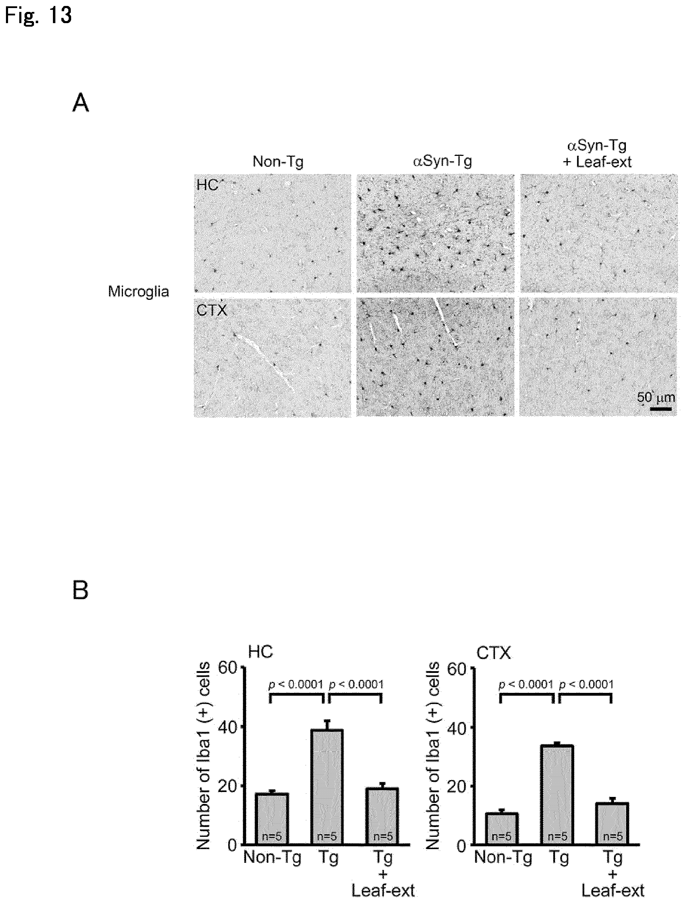

[Figure 13] Figure 13 shows microglia pathology in the hippocampus (HC) and cerebral cortex (CTX) of Huα-Syn (A53T) mice administered ma̅maki leaf hot water extract (Leaf-ext) at a dose of 100 µg/day orally for one month (labeled "αSyn-Tg + Leaf-ext" in the table), in comparison with Huα-Syn (A53T) mice administered water (labeled "αSyn-Tg" in the table) and non-genetically modified mice (labeled "Non-Tg" in the table). Fig. 13A shows photographs of the staining results for activated microglia, and Fig. 13B shows graphs of the number of positive cells in each photograph.

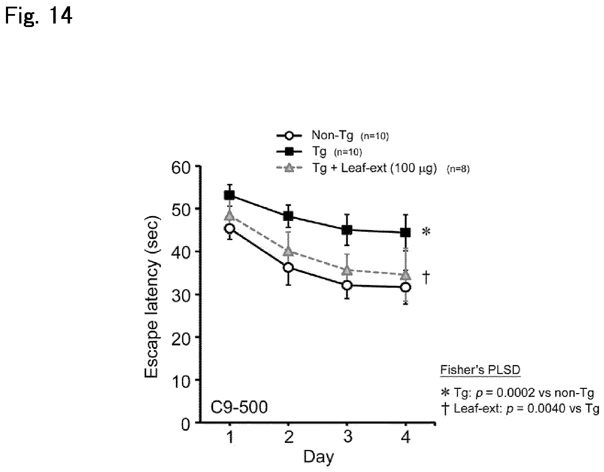

[Figure 14] Figure 14 is a graph showing the results of the Morris water maze test for C9-500 mice administered ma̅maki leaf hot water extract (Leaf-ext) at a dose of 100 µg/day orally for one month (labeled "Tg + Leaf-ext" in the table), in comparison with C9-500 mice administered water (labeled "Tg" in the table) and non-genetically modified mice (labeled "Non-Tg" in the table).

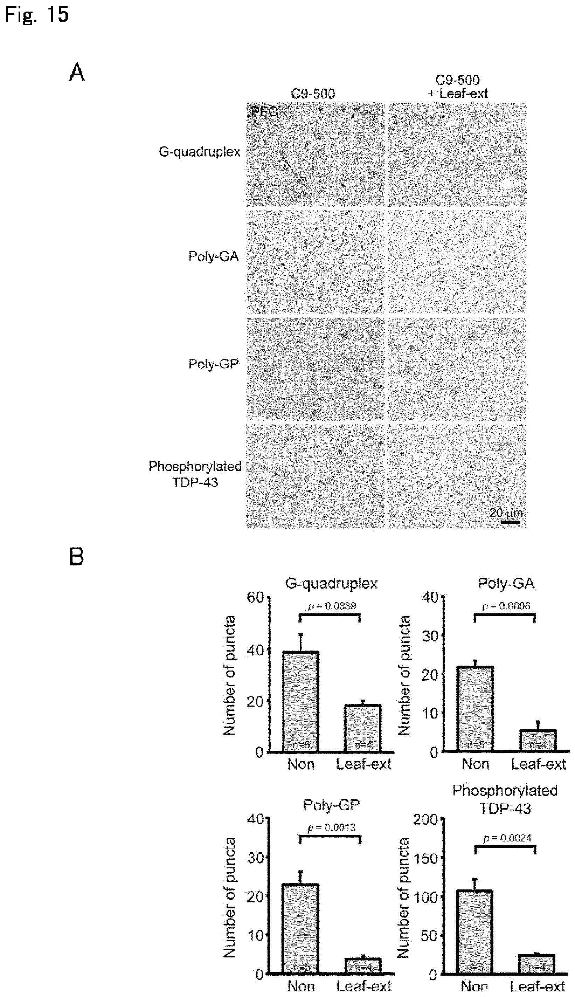

[Figure 15] Figure 15 shows pathology resulting from a mutation in the C9orf72 gene in the prefrontal cortex (PFC) of C9-500 mice administered ma̅maki leaf hot water extract (Leaf-ext) at a dose of 100 µg/day orally for one month (labeled "C9-500 + Leaf-ext" in the table), in comparison with C9-500 mice administered water (labeled "C9-500" in the table). Fig. 15A shows photographs of the staining results for RNA G-quadruplex, poly-GA, poly-GP, and phosphorylated TDP-43, and Fig. 15B shows graphs of the quantitative values of the staining intensity in each photograph.

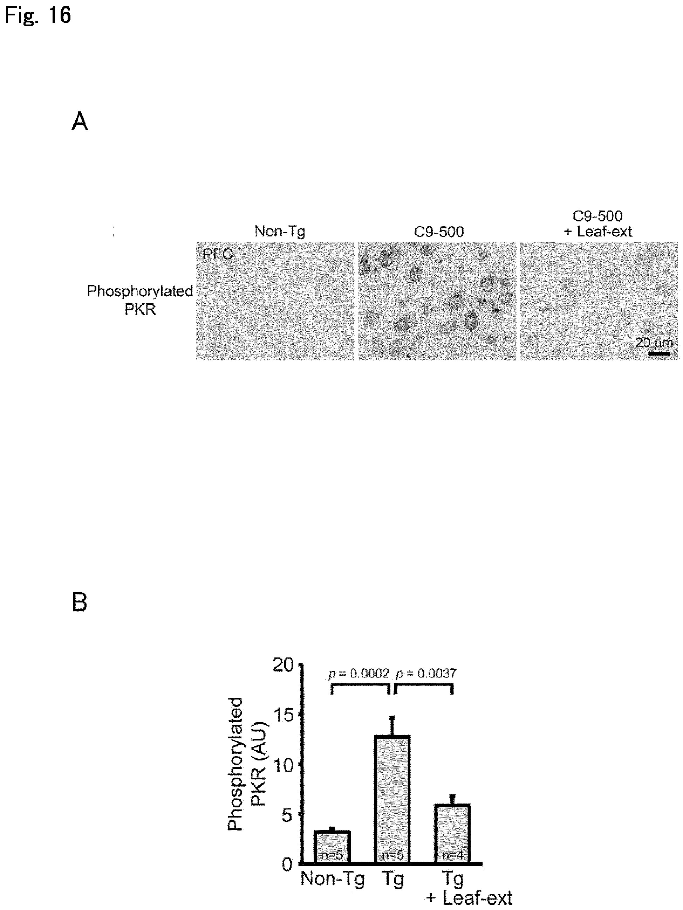

[Figure 16] Figure 16 shows double-stranded RNA-dependent protein kinase (PKR) pathology in the prefrontal cortex (PFC) of C9-500 mice administered ma̅maki leaf hot water extract (Leaf-ext) at a dose of 100 µg/day orally for one month (labeled "C9-500 + Leaf-ext" in the table), in comparison with C9-500 mice administered water (labeled "C9-500" in the table) and non-genetically modified mice (labeled "Non-Tg" in the table). Fig. 16A shows photographs of the staining results for phosphorylated PKR, and Fig. 16B shows graphs of the quantitative values of the staining intensity in each photograph.

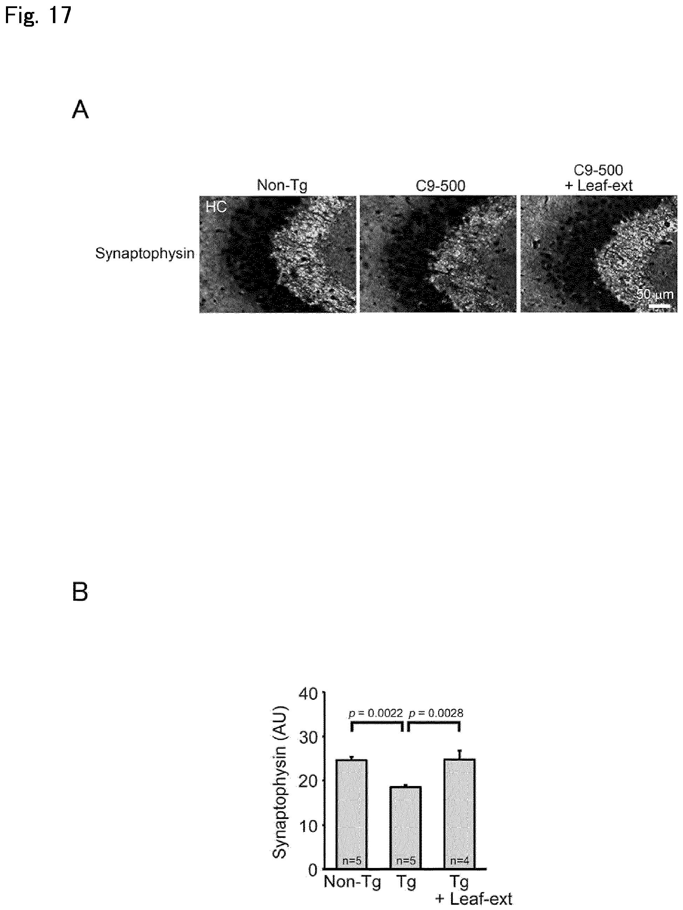

[Figure 17] Figure 17 shows synaptophysin pathology in the hippocampal CA2/3 region of C9-500 mice administered ma̅maki leaf hot water extract (Leaf-ext) at a dose of 100 µg/day orally for one month (labeled "C9-500 + Leaf-ext" in the table), in comparison with C9-500 mice administered water (labeled "C9-500" in the table) and non-genetically modified mice (labeled "Non-Tg" in the table). Fig. 17A shows photographs of the staining results for synaptophysin, and Fig. 17B shows graphs of the quantitative values of the staining intensity in each photograph.

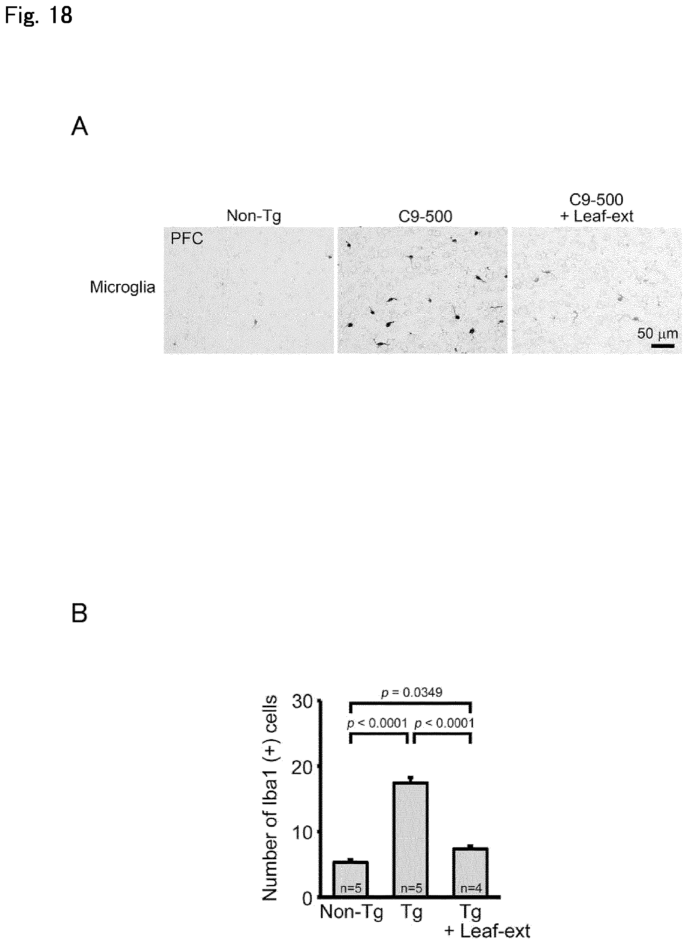

[Figure 18] Figure 18 shows microglia pathology in the prefrontal cortex (PFC) of C9-500 mice administered ma̅maki leaf hot water extract (Leaf-ext) at a dose of 100 µg/day orally for one month (labeled "C9-500 + Leaf-ext" in the table), in comparison with C9-500 mice administered water (labeled "C9-500" in the table) and non-genetically modified mice (labeled "Non-Tg" in the table). Fig. 18A shows photographs of the staining results for activated microglia, and Fig. 18B shows graphs of the number of positive cells in each photograph.

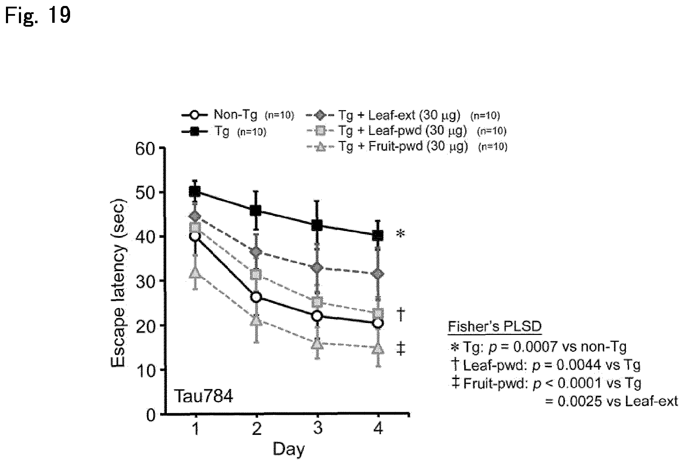

[Figure 19] Figure 19 is a graph showing the results of the Morris water maze test for Tau784 mice administered ma̅maki leaf hot water extract (Leaf-ext), ma̅maki leaf simple crushed powder (Leaf-pwd), and ma̅maki fruit (including seeds) simple crushed powder (Fruit-pwd) at a dose of 30 µg/day orally for one month (labeled "Tg + Leaf-ext," "Tg + Leaf-pwd," and "Tg + Fruit-pwd" respectively in the table), in comparison with Tau784 mice administered water (labeled "Tg" in the table) and non-genetically modified mice (labeled "Non-Tg" in the table).

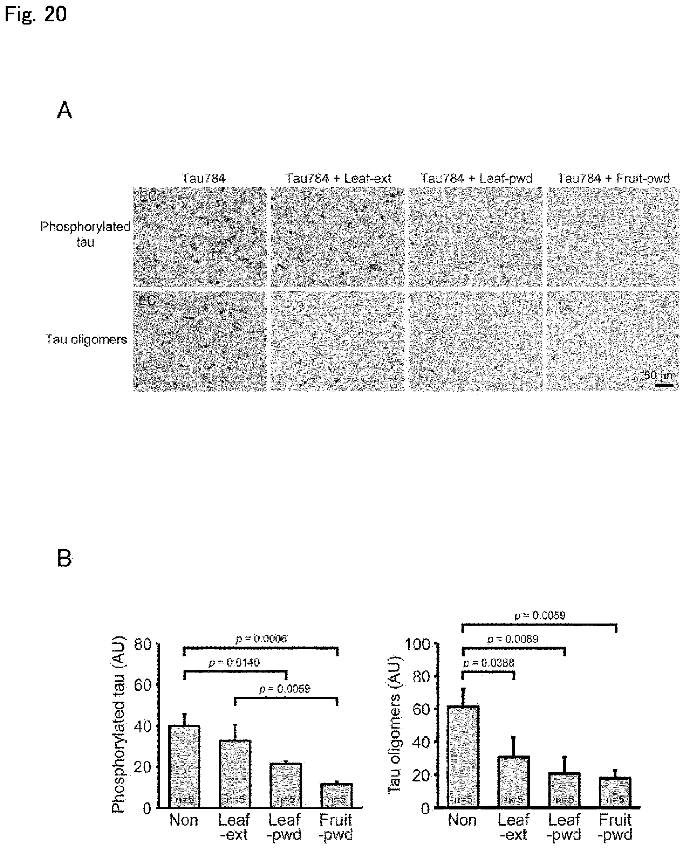

[Figure 20] Figure 20 shows tau pathology in the entorhinal cortex of Tau784 mice administered ma̅maki leaf hot water extract (Leaf-ext), ma̅maki leaf simple crushed powder (Leaf-pwd), and ma̅maki fruit (including seeds) simple crushed powder (Fruit-pwd) at a dose of 30 µg/day orally for one month (labeled "Tg + Leaf-ext," "Tg + Leaf-pwd," and "Tg + Fruit-pwd" respectively in the table), in comparison with Tau784 mice administered water (labeled "Tau784" in the table). Fig. 20A shows photographs of the staining results for phosphorylated tau and tau oligomers, and Fig. 20B shows graphs of the quantitative values of the staining intensity in each photograph.

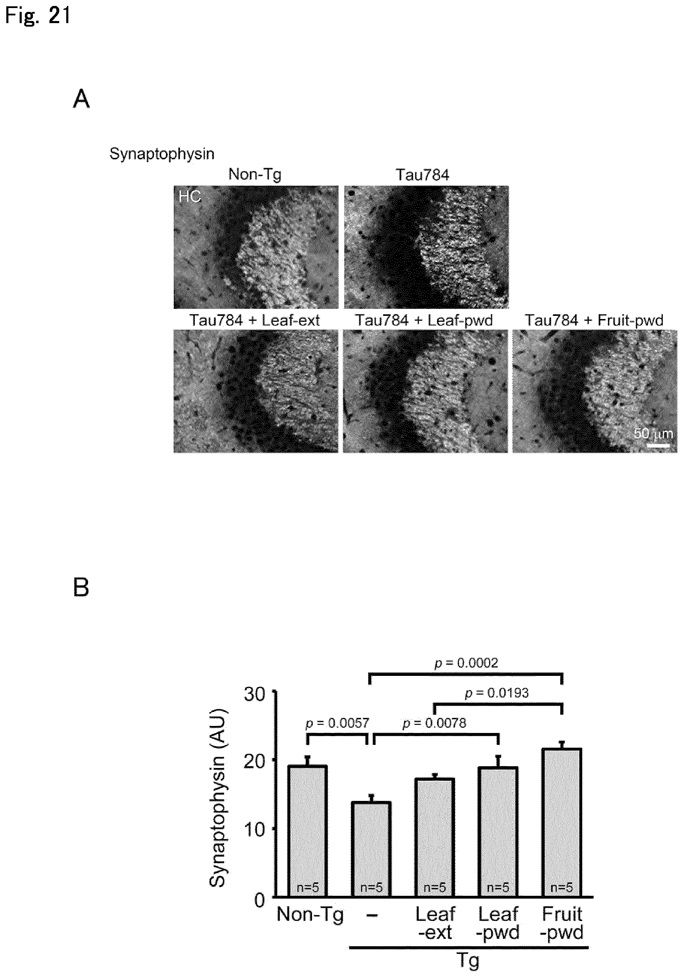

[Figure 21] Figure 21 shows synaptophysin pathology in the hippocampal CA2/3 region of Tau784 mice administered ma̅maki leaf hot water extract (Leaf-ext), ma̅maki leaf simple crushed powder (Leaf-pwd), and ma̅maki fruit (including seeds) simple crushed powder (Fruit-pwd) at a dose of 30 µg/day orally for one month (labeled "Tg + Leaf-ext," "Tg + Leaf-pwd," and "Tg + Fruit-pwd" respectively in the table), in comparison with Tau784 mice administered water (labeled "Tau784" in the table) and non-genetically modified mice (labeled "Non-Tg" in the table). Fig. 21A shows photographs of the staining results for synaptophysin, and Fig. 21B shows graphs of the quantitative values of the staining intensity in each photograph.

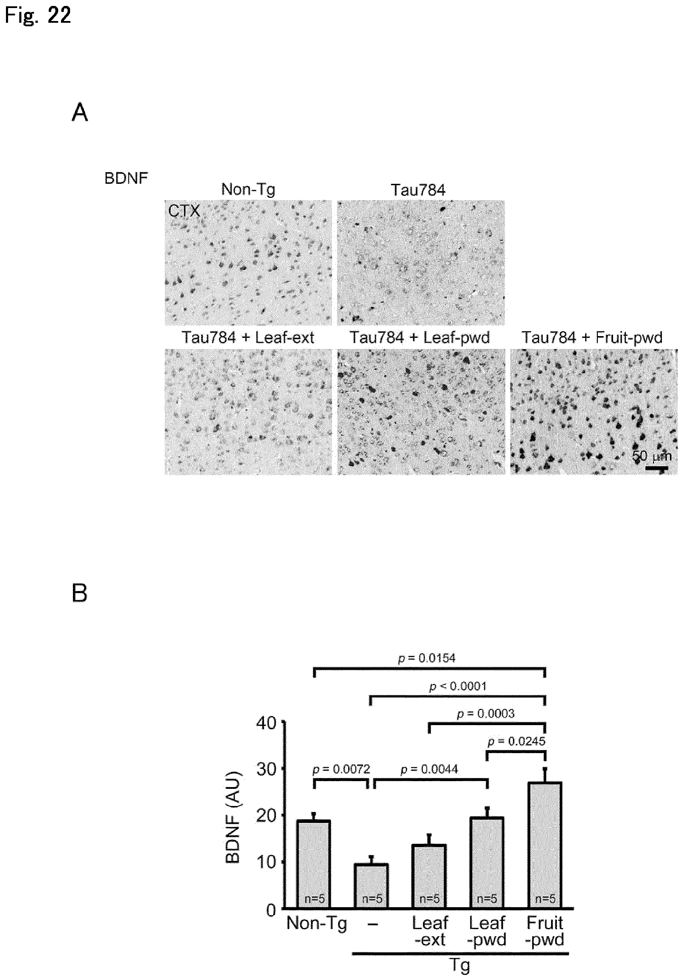

[Figure 22] Figure 22 shows BDNF expression in the cerebral cortex (CTX) of Tau784 mice administered ma̅maki leaf hot water extract (Leaf-ext), ma̅maki leaf simple crushed powder (Leaf-pwd), and ma̅maki fruit (including seeds) simple crushed powder (Fruit-pwd) at a dose of 30 µg/day orally for one month (labeled "Tg + Leaf-ext," "Tg + Leaf-pwd," and "Tg + Fruit-pwd" respectively in the table), in comparison with Tau784 mice administered water (labeled "Tau784" in the table) and non-genetically modified mice (labeled "Non-Tg" in the table). Fig. 22A shows photographs of the staining results for BDNF, and Fig. 22B shows graphs of the quantitative values of the staining intensity in each photograph.

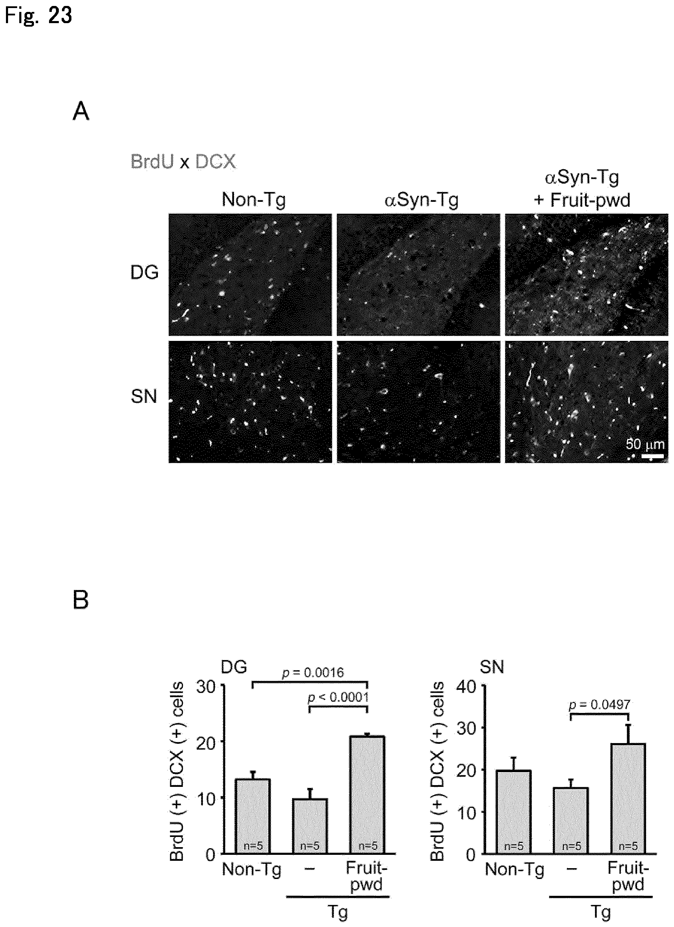

[Figure 23] Figure 23 shows neurogenesis levels in the dentate gyrus (DG) and substantia nigra (SN) of Huα-Syn (A53T) mice administered ma̅maki fruit (including seeds) simple crushed powder (Fruit-pwd) at a dose of 30 µg/day orally for one month (labeled "αSyn-Tg + Fruit-pwd" in the table), in comparison with Huα-Syn (A53T) mice administered water (labeled "αSyn-Tg" in the table) and non-genetically modified mice (labeled "Non-Tg" in the table). Fig. 23A shows immunofluorescence staining photographs for BrdU (red) and doublecortin (DCX) (green). Double-positive cells (yellow), which are positive for both BrdU (red) and DCX (green), were regarded as newborn neurons. Fig. 23B shows graphs of the quantitative results of counting the number of double-positive cells (yellow) in each photograph.

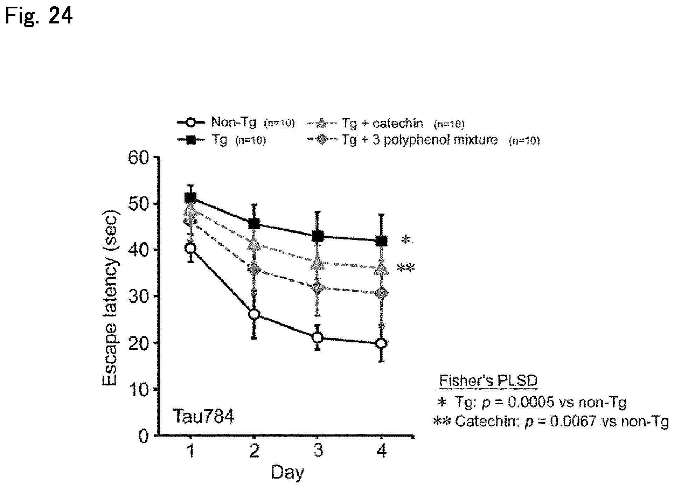

[Figure 24] Figure 24 is a graph showing the results of the Morris water maze test for Tau784 mice administered a mixture of catechin at 0.087 µg, chlorogenic acid at 0.036 µg, and rutin at 0.123 µg (labeled "Tg + 3 polyphenol mixture" in the table), or catechin only at 0.087 µg (labeled "Tg + catechin" in the table), orally for one month, in comparison with Tau784 mice administered water (labeled "Tg" in the table) and non-genetically modified mice (labeled "Non-Tg" in the table).

### EMBODIMENTS OF THE INVENTION

Hereinafter, the present invention will be described in detail with reference to specific embodiments. However, the present invention is not limited to these embodiments and can be implemented in any form without departing from the scope of the present invention.

### [Summary]

As mentioned above, aside from issues related to side effects, the primary reasons why most of the anti-dementia drugs that have been studied to date have failed are believed to be the delayed timing of drug administration and targeting the wrong molecules. It has been shown that Aβ accumulation in the brain begins more than 20 years before the onset of Alzheimer's disease, and tau accumulation begins approximately 10 years prior. As Aβ and tau accumulate and neurons begin to die, dementia manifests. In other words, by the time dementia becomes apparent, a significant number of neurons have already died. If the goal is to remove Aβ or tau, it must be done before neurons begin to die. That is, the role of drugs targeting Aβ or tau is preventive, not therapeutic. Previously, it was thought that the disease arose when insoluble protein aggregates, such as senile plaques or neurofibrillary tangles (accumulations of aggregated tau), killed neurons. However, recent research suggests that soluble oligomers, which form at an earlier stage, impair neuronal function and lead to the onset of dementia. Therefore, it is necessary to remove these oligomers to prevent dementia.

From a preventive perspective, it would be preferable for a single drug to act on various causative protein oligomers rather than being specific to Aβ, tau, or α-synuclein. Additionally, damaged nerve cells must be repaired and brain function restored. Furthermore, since preventing dementia is a long-term process, preventive medications should be safe and affordable. Ideally, they should also be self-administered non-invasively, without the need for medical assistance. Given the numerous requirements for dementia preventive medications, it is difficult to achieve them with a single-component pharmaceutical. Furthermore, if all middle-aged and older adults were to take these medications long-term to prevent dementia, it could eventually lead to the collapse of the healthcare economy.

To solve these problems, the present inventors have focused on herbal medicines, which have been used in traditional Chinese and Kampo medicine with a long history. If effective herbal medicines for improving cognitive function existed, middle-aged and elderly people could buy them without consulting a doctor and add them to their diet for consumption, whereby they could work to prevent dementia in their daily lives. Based on this idea, the present inventors have conducted intensive research and found that an herbal medicine selected from ma̅maki leaves, fruit, and seeds has various effects such as maintaining or improving cerebral function, promoting nerve cell repair or inducing neurogenesis, removing a neurodegenerative disease-causing protein that accumulates in the brain, or treating or preventing a neurodegenerative disease. The present invention is based on these findings.

Thus, one aspect of the present invention provides an agent for maintaining or improving a cerebral function, an agent for promoting nerve cell repair or inducing neurogenesis, an agent for removing a neurodegenerative disease-causing protein that accumulates in the brain, or an agent for treating or preventing a neurodegenerative disease, each containing an herbal medicine selected from ma̅maki leaves, fruit, and seeds (these may be collectively referred to as an "agent of the present invention").

Another aspect of the present invention provides a food product containing an agent of the present invention (this may be referred to as a "food product of the present invention").

Yet another aspect of the present invention provides a pharmaceutical product containing an agent of the present invention (this may be referred to as a "pharmaceutical product of the present invention").

Still another aspect of the present invention provides a method for maintaining or improving a cerebral function, a method for promoting nerve cell repair or inducing neurogenesis, a method for removing a neurodegenerative disease-causing protein that accumulates in the brain, or a method for treating or preventing a neurodegenerative disease (these may be collectively referred to as a "method of the present invention"), the method comprising administering one or more of an herbal medicine selected from ma̅maki leaves, fruit, and seeds, the agent of the present invention, the food product of the present invention, and the pharmaceutical product of the present invention to a subject.

### [Ma̅maki]

According to one embodiment, the agent of the present invention contains an herbal medicine selected from ma̅maki leaves, fruit, and seeds. As mentioned above, the leaves of the Hawaiian native herb ma̅maki (*Pipturus albidus*) are used as a popular herbal tea ingredient in Hawaii. In Hawaii, ma̅maki, especially its fruit, has a history of being used as a natural folk remedy to regulate blood sugar, blood pressure, and cholesterol levels, relieve stress and fatigue, and reduce inflammation (Non-Patent Literature 9: Rafii et al., Alzheimers Dement., (2022), 1-7). However, its effect on dementia is not known.

The main components identified in ma̅maki leaves are three polyphenols: (+)-catechin, chlorogenic acid, and rutin (Non-Patent Literature 10: Chun et al., Native Hawaiian medicines, First People's Productions Honolulu, (1994), 216-217). The content of catechin and rutin in ma̅maki leaves is significantly higher than in other commercially available tea leaves (Non-Patent Literature 10). These polyphenols have been suggested to have beneficial effects on model mice and humans with neurodegenerative diseases.

Catechins are a type of flavonoid, consisting of catechin itself and its derivatives, and are abundant in tea leaves. They show anti-inflammatory and antioxidant effects by blocking cytokine production and inflammatory pathways, chelating metal ions, and scavenging free radicals (Non-Patent Literature 11: Afzal et al., Molecules, (2022), 27[21]:7604; and Non-Patent Literature 12: Payne et al., Biomolecules, (2022), 12[3]:371). Catechins, especially epigallocatechin-3-gallate, have been shown to inhibit Aβ production, tau phosphorylation, and amyloid protein aggregation, thereby preventing cognitive decline in AD and PD (Non-Patent Literature 11 and 12). Furthermore, it has also been reported that oral administration to mice increases the expression of brain-derived neurotrophic factor (BDNF) and neurogenesis (Non-Patent Literature 13: Li et al., Neuroscience, (2009), 159[4]:1208-15; and Non-Patent Literature 14: Yoo et al., Phytother Res., (2010), 24[7]:1065-70). BDNF and neurogenesis are important for brain repair and regeneration (Non-Patent Literature 15: Colucci-D'Amato et al., Int. J. Mol. Sci., (2020), 21[20]:7777; and Non-Patent Literature 16: Horgusluoglu et al., Am. J. Med. Gene. B. Neuropsychiatr. Genet., (2017), 174[1]:93-112).

Chlorogenic acid, also known as 5-O-caffeoylquinic acid, is abundant in coffee beans. It has a wide range of beneficial health effects, including anti-inflammatory, antioxidant, neuroprotective, hepatoprotective, cardioprotective, chemopreventive, anti-diabetic, and anti-obesity effects, and regular intake has been suggested to reduce the risk of neurodegenerative diseases and improve cognitive function (Non-Patent Literature 17: Socala et al., Int. J. Mol. Sci., (2020), 22[1]:107). Oral administration to mouse models of AD and PD improves their memory and motor function (Non-Patent Literature 18: Gao et al., Drug Des. Devel. Ther., (2020), 14:1705-1716; and Non-Patent Literature 19: Singh et al., Oxid. Med. Cell Longev., (2020), 2020:6571484), and oral administration to a rat model of cerebral ischemia/reperfusion has been shown to promote the expression of BDNF and nerve growth factor (NGF) (Non-Patent Literature 20: Liu et al., Drug Des. Devel. Ther., (2020), 14:51-60).

Rutin is a member of the flavonol subtype of flavonoids and is found in various plants. It exhibits anti-inflammatory, antioxidant, and neuroprotective effects (Non-Patent Literature 21: Tahir et al., Biomed. Pharmacother., (2021), 137:111253). Oral administration to AD model mice reduces Aβ and tau oligomers and improves cognitive function (Non-Patent Literature 22: Xu et al., Behav. Brain Res., (2014), 264:173-80; and Non-Patent Literature 23: Sun et al., J. Neuroinflammation, (2021), 18[1]:131). Furthermore, it has also been reported that intraperitoneal administration to rats with Aβ injected into the brain increases BDNF expression (Non-Patent Literature 24: Moghbelinejad et al., Toxicol Lett., (2014), 224[1]:108-13).

As described above, the three polyphenols contained in ma̅maki leaves-catechin, chlorogenic acid, and rutin-have been suggested to have effects such as improving cognitive function. However, the effects of ma̅maki leaves and fruit on cognitive function improvement are not known at all.

Therefore, as will be detailed in the examples below, the present inventors separated leaves and fruit (including seeds) from commercially available dried ma̅maki tea leaves, prepared hot water extracts and simple crushed powders of each, and investigated their effects on cognitive function and neuropathology using four types of degenerative dementia model mice.

First, using Tau784 mice (Non-Patent Literature 25: Umeda et al., Am. J. Pathol., (2013), 183[1]:211-25; and Non-Patent Literature 26: Umeda et al., Ann. Clin. Transl. Neurol., (2015), 2[3]:241-55) as a model of frontotemporal dementia-tau (FTD-Tau), ma̅maki leaf hot water extract (Leaf-ext) was administered for one month at doses of 1000 µg/day, 100 µg/day, and 30 µg/day (Example 1). As a result, significant improvements were observed in cognitive function (Fig. 1), tau pathology (Fig. 2), synaptophysin pathology (Fig. 3), and microglia pathology (Fig. 4) at all doses. In particular, when administered at doses of 1000 µg/day and 100 µg/day, each pathology was improved to a level equivalent to that of non-genetically modified littermates.

Next, using APP23 mice (Non-Patent Literature 27: Sturchler-Pierrat et al., Proc. Natl. Acad. Sci. U.S.A., (1997), 94[24]:13287-92; Non-Patent Literature 28: Van Dam et al., Eur. J. Neurosci., (2003), 17[2]:388-96; and Non-Patent Literature 29: Umeda et al., Front. Neurosci., (2021), 15:763476) as a model of Alzheimer's disease (AD), ma̅maki leaf hot water extract (Leaf-ext) was administered at a dose of 100 µg/day for one month (Example 2). As a result, each pathology, including cognitive function (Fig. 5), amyloid pathology (Fig. 6), synaptophysin pathology (Fig. 7), and microglia pathology (Fig. 8), was improved to a level equivalent to or close to that of non-genetically modified littermates.

Furthermore, using Huα-Syn (A53T) mice (Non-Patent Literature 30: Lee et al., Proc. Natl. Acad. Sci. U.S.A., (2002), 99[13]:8968-73; and Non-Patent Literature 31: Umeda et al., Int. J. Mol. Sci., (2021), 22:8453) as a model of Lewy body dementia (DLB), ma̅maki leaf hot water extract (Leaf-ext) was administered at a dose of 100 µg/day for one month (Example 3). As a result, each pathology, including cognitive function (Fig. 9), synuclein pathology (Figs. 10 and 11), synaptophysin pathology (Fig. 12), and microglia pathology (Fig. 13), was improved to a level equivalent to or close to that of non-genetically modified littermates.

Additionally, using C9-500 mice (Non-Patent Literature 32: Liu et al., Neuron, (2016), 90[3]:521-534; and Non-Patent Literature 33: Hatanaka et al., Biomedicines, (2022), 10[5]:1080) as a model of frontotemporal dementia-TDP (FTD-TDP), ma̅maki leaf hot water extract (Leaf-ext) was administered at a dose of 100 µg/day for one month (Example 4). As a result, each pathology, including cognitive function (Fig. 14), pathology resulting from a mutation in the C9orf72 gene (Fig. 15), double-stranded RNA-dependent protein kinase (PKR) pathology (Fig. 16), synaptophysin pathology (Fig. 17), and microglia pathology (Fig. 18), was improved to a level equivalent to or close to that of non-genetically modified littermates. The above results suggest that ma̅maki leaves have the effect of improving cognitive function and various related pathologies.

Furthermore, using Tau784 mice (Non-Patent Literature 25 and 26, mentioned above) as a model of frontotemporal dementia-tau (FTD-Tau), ma̅maki leaf hot water extract (Leaf-ext), ma̅maki leaf simple crushed powder (Leaf-pwd), and ma̅maki fruit (including seeds) simple crushed powder (Fruit-pwd) were each administered at a dose of 30 µg/day for one month (Example 5). As a result, significant improvements were observed for all preparations in cognitive function (Fig. 19), tau pathology (Fig. 20), synaptophysin pathology (Fig. 21), and BDNF expression (Fig. 22). In particular, ma̅maki leaf simple crushed powder (Leaf-pwd) and ma̅maki fruit (including seeds) simple crushed powder (Fruit-pwd) improved each pathology to a level equivalent to or close to that of non-genetically modified littermates. Surprisingly, with ma̅maki fruit (including seeds) simple crushed powder (Fruit-pwd), the cognitive function of the Tau784 mice was significantly enhanced to a level even higher than that of non-genetically modified littermates. The above results suggest that crushed products of ma̅maki leaves and fruit (including seeds) have a much better effect on improving cognitive function and various pathologies than extracts.

Moreover, using Huα-Syn (A53T) mice (Non-Patent Literature 30 and 31, mentioned above) as a model of Lewy body dementia (DLB), ma̅maki fruit (including seeds) simple crushed powder (Fruit-pwd) was administered at a dose of 100 µg/day for one month (Example 6). As a result, the neurogenesis levels in the dentate gyrus (DG) and substantia nigra (SN) of the Huα-Syn (A53T) mice were, surprisingly, improved to a level far exceeding that of non-genetically modified littermates. The above results suggest that crushed products of ma̅maki fruit (including seeds) have the effect of promoting brain rejuvenation through neuronal repair and regeneration.

To verify whether the above-mentioned effects of ma̅maki leaves and fruit (including seeds) in improving cognitive function and various pathologies are due to the three polyphenols (catechin, chlorogenic acid, and rutin) contained in ma̅maki leaves, the following experiment was conducted. Specifically, using Tau784 mice (Non-Patent Literature 25 and 26, mentioned above) as a model of frontotemporal dementia-tau (FTD-Tau), a mixture of catechin, chlorogenic acid, and rutin corresponding to the amount contained in 30 µg of ma̅maki fruit (including seeds) simple crushed powder, or catechin alone, was administered for one month. As a result, the effect of this catechin/chlorogenic acid/rutin mixture and catechin alone on improving cognitive function was far inferior to that of non-genetically modified littermates and was incomplete. Therefore, it is understood that the various effects of ma̅maki leaf hot water extract (Leaf-ext), ma̅maki leaf simple crushed powder (Leaf-pwd), and ma̅maki fruit (including seeds) simple crushed powder (Fruit-pwd) are not caused solely by the three polyphenols, but rather that other unknown components contained in ma̅maki contribute significantly.

### [Form of herbal medicines]

According to one embodiment, ma̅maki leaves, fruit, and seeds can be used alone or in combination. Specifically, ma̅maki leaves can be used alone, ma̅maki fruit can be used alone, ma̅maki seeds can be used alone, or any two or all three of ma̅maki leaves, fruit, and seeds can be used in combination.

Ma̅maki leaves and/or fruit and/or seeds can be used in any form. Examples of such forms include, but are not limited to, using ma̅maki leaves and/or fruit and/or seeds as they are, or in the form of a crushed product and/or an extract.

Specifically, when an herbal medicine selected from ma̅maki leaves, fruit, and seeds is in the form of a crushed product, such as crushed powder, the processing conditions are not particularly limited, but are, for example, as follows. First, the herbal medicine is dried. The drying conditions are not limited, and various known conditions may be used. Examples include natural drying and heat drying at a temperature of 0 to 100°C. Next, the dried herbal medicine is crushed. The means of crushing are not limited, and various known methods may be used, including manual crushing and crushing using a pulverizer such as a wet crushing method or a dry crushing method. The crushed product of the herbal medicine obtained can be used as is, or a powder with a particle size controlled to a predetermined value or less can be obtained by sieving. For example, a sieve with a mesh size of 0.02 mm to 20 mm can be used to adjust the particle size.

When an herbal medicine selected from ma̅maki leaves, fruit, and seeds is in the form of an extract, the processing conditions are not particularly limited. For example, extraction can be performed by adding an extraction solvent after arbitrarily drying and/or crushing the herbal medicine. Specifically, extraction can be performed by adding, for example, 1 to 100 parts by mass of an extraction solvent to 1 part by mass of the dried crushed herbal medicine and holding it for, for example, 0.1 to 100 hours. The extraction solvent is not particularly limited, and various known solvents can be used. Examples include water and hydrophilic organic solvents such as ethanol. Extraction using an extraction solvent can be carried out at ordinary temperature or elevated temperature. For example, when carried out with water under heating, the crushed product can be heated at 30 to 100°C with the addition of hot water. If necessary, stirring, ultrasonic treatment, heating reflux treatment, etc., can be used in combination to increase the extraction efficiency. After extraction is complete, the liquid and solid components are separated manually or by filtration. The separated liquid component can be used as the extract. In any case, the solvent may be evaporated and concentrated before use, if necessary.

### [Effects of the agents]

According to one embodiment, the agent of the present invention is for maintaining or improving a cerebral function. The statement "maintaining or improving a cerebral function" herein refers to, but is not limited to, maintaining or improving cognitive function and/or motor function.

The statement "maintaining or improving cognitive function" herein refers to, but is not limited to, preventing the occurrence of or improving abnormalities such as mild forgetfulness, memory impairment seen in dementia, disorientation, impaired judgment and understanding, executive dysfunction, apraxia, agnosia, and aphasia.

The statement "maintaining or improving motor function" herein refers to, but is not limited to, preventing the occurrence of or improving abnormalities such as tremor, muscle rigidity, postural reflex disorders, akinesia/bradykinesia, and other Parkinsonian symptoms seen in Parkinson's disease; involuntary movements seen in Huntington's disease; coordination disorders seen in spinocerebellar degeneration; and muscle weakness seen in amyotrophic lateral sclerosis.

The effects of maintaining or improving a cerebral function by the agent of the present invention can be evaluated, for example, as mentioned in the Examples described below, by administering the agent to model animals that develop brain pathology and then subjecting the animals to a cognitive function test such as the Morris water maze test and/or a motor function test such as the rotarod test, or by staining and observing brain sections of the animals.

According to one embodiment, the agent of the present invention is for promoting nerve cell repair or inducing neurogenesis. The statement "promoting nerve cell repair" herein may refer to, but is not limited to, promoting the recovery of the number and function of synapses that have decreased, promoting the recovery of the function of nerve cells that have decreased, promoting the maintenance and recovery of the function of synapses and nerve cells, and promoting the expression of brain-derived neurotrophic factor (BDNF) and nerve growth factor (NGF), which protect nerve cells from various stresses. The statement "inducing neurogenesis" herein may refer to, but is not limited to, inducing the generation of new immature nerve cells that actively synthesize DNA. Indicators of DNA synthesis include the active uptake of exogenously administered nucleic acid analogues such as BrdU and the expression of markers of immature neurons such as doublecortin. The effects of promoting nerve cell repair or inducing neurogenesis by the agent of the present invention can be evaluated, for example, as mentioned in the Examples described below, by administering the agent to model animals that develop dementia or brain pathology and then observing the induction of brain-derived neurotrophic factor (BDNF) expression in the animals or by staining and observing brain sections of the animals to observe new neurons.

According to one embodiment, the agent of the present invention is for removing a neurodegenerative disease-causing protein that accumulates in the brain. The term "neurodegenerative disease-causing protein" herein refers to a protein that has been identified or presumed to be a cause of dementia. Examples include, but are not limited to, one or more proteins selected from amyloid β (Aβ), tau, α-synuclein, TDP-43, FUS/TLS, polyglutamine, a protein resulting from RAN (repeat-associated non-ATG) translation, prion, and SOD-1. The effects of maintaining or improving a cerebral function by the agent of the present invention can be evaluated, for example, as mentioned in the Examples described below, by administering the agent to model animals that develop dementia and then staining and observing brain sections of the animals.

According to one embodiment, the agent of the present invention is for treating or preventing a neurodegenerative disease. The term "neurodegenerative disease" herein refers to, but is not limited to, a neurodegenerative disease in which any of the neurodegenerative disease-causing proteins mentioned above accumulate in the brain. Examples of neurodegenerative diseases include, but are not limited to, Alzheimer's disease (Aβ and tau), frontotemporal lobar degeneration (tau, TDP-43, and FUS/TLS), Lewy body dementia (α-synuclein), Parkinson's disease (α-synuclein), multisystem atrophy (α-synuclein), Huntington's disease (polyglutamine), amyotrophic lateral sclerosis (TDP-43, FUS/TLS, RAN protein, and SOD-1), spinal cord cerebellum degeneration (polyglutamine, RAN protein), and Creutzfeldt-Jakob disease (prion) (examples of neurodegenerative disease-causing proteins that accumulate in each neurodegenerative disease are shown in parentheses). Among these, the agent for treating or preventing a neurodegenerative disease of the present invention may preferably be an agent for treating or preventing degenerative dementia. Examples of degenerative dementia include, but are not limited to, Alzheimer's disease, frontotemporal dementia, Lewy body dementia, and dementia resulting from Parkinson's disease or amyotrophic lateral sclerosis. Among these, frontotemporal dementia, which is characterized by the accumulation of tau, can be further classified into Pick's disease, corticobasal degeneration, and progressive supranuclear palsy, for example. The effect of treating or preventing a neurodegenerative disease by the agent of the present invention can be evaluated, for example, as mentioned in the Examples described below, by administering the agent to model animals that develop a neurodegenerative disease (e.g., degenerative dementia) and then subjecting the animals to a cognitive function test such as the Morris water maze test and a motor function test such as the rotarod test, or by staining and observing brain sections of the animals.

### [Form, use, and dosage of the agents]

The form of the agent of the present invention is not particularly limited. For example, an herbal medicine selected from ma̅maki leaves, fruit, and seeds can be used as it is in any form, such as a crushed product, an extract of the crushed product, or an extraction residue of the crushed product, or it can be formulated for use in combination with other ingredients such as desired excipients and/or carriers. When used in the form of a food product (the food product of the present invention) or a pharmaceutical product (the pharmaceutical product of the present invention) containing the agent of the present invention, the ma̅maki leaves and/or fruit and/or seeds, which are the active ingredients of the agent of the present invention, can be mixed with other ingredients appropriate for the form of the food or pharmaceutical product for use. Details will be described later.

The method of use of the agent of the present invention is not particularly limited, but it is usually administered orally. In particular, the agent of the present invention is preferably used in the form of food (the food product of the present invention) or an oral drug (the pharmaceutical product of the present invention). Details will be described later.

The dosage of the agent of the present invention is not particularly limited. For example, the agent of the present invention can be administered to a subject in an amount such that the dosage of the active ingredients, i.e., ma̅maki leaves and/or fruit and/or seeds, may usually be 0.01 mg/day or more, or 0.1 mg/day or more, preferably 0.5 mg/day or more, more preferably 1.0 mg/day or more, and usually 10 g/day or less, preferably 5 g/day or less, and further preferably 1 g/day or less.

### [Food product]

One aspect of the present invention provides a food product containing an agent of the present invention (the food product of the present invention). The food product of the present invention is characterized by containing any one of the agents of the present invention and is used for the purpose of the agent.

The food product of the present invention can be in any form that can be ingested orally, such as a solution, suspension, emulsion, powder, or solid molded product. In addition, as with the pharmaceuticals of the present invention described below, it can be made into dosage forms such as capsules, lozenges, syrups, and granules.

The food product of the present invention can be manufactured as, for example, beverages such as tea, black tea, coffee, soft drinks, alcoholic beverages, carbonated beverages, milk beverages, fruit juice beverages, nutritional drinks, concentrated beverages, and powdered beverages (powdered juice, powdered soup, etc.); supplements; confectioneries such as candy, gummy candy, gum, chocolate, cookies, and biscuits; frozen desserts such as ice cream; dairy products such as yogurt and processed milk; flour products such as cereals, bread, and cake mix; noodles such as soba; processed oil and fat products such as mayonnaise, whipped cream, and salad dressing; processed marine products; processed livestock products; and processed agricultural products. The food product of the present invention can be produced by adding the agent of the present invention during the production of these food products.

In addition to other food ingredients, the food product of the present invention may contain, as needed, various additives such as: sweeteners, colorants, preservatives, thickening agents, stabilizers, gelling agents or pasting agents, antioxidants such as ascorbic acid, color developers, bleaching agents, mold inhibitors or fungicides, yeast food, gum bases, alkaline agents, bittering agents, enzymes, glossing agents, flavorings, acidulants, chewing gum softeners, seasonings, tofu coagulants, emulsifiers, pH adjusters, leavening agents, vitamins, minerals, amino acids, and other nutritional fortifiers, as well as manufacturing aids.

The food product of the present invention may be provided as a food product that indicates the action, effects, functions, or uses of the agent of the present invention under the system of each country. For example, in Japan, the food products of the present invention may be manufactured as health functional foods (foods with specified health functions, foods with functional claims, and foods with nutritional functions).

The content of the active ingredients of the agent of the present invention, i.e., ma̅maki leaves and/or fruit and/or seeds, in the food product of the present invention can be set appropriately within the range in which the effect of the present invention can be obtained, as with the agent of the present invention. Specifically, the content of the active ingredients of the agent of the present invention, i.e., ma̅maki leaves and/or fruit and/or seeds, may be adjusted to within the range of usually 0.01 mg/day or more, or 0.1 mg/day or more, preferably 0.5 mg/day or more, more preferably 1.0 mg/day or more, and usually 10 g/day or less, preferably 5 g/day or less, more preferably 1 g/day or less.

The number of times and frequency of intake of the food product of the present invention are arbitrary and can be set as appropriate at any number of times and frequency, such as once to several times a day, every day, every other day, every two days, or once to seven times a week. By combining the desired amount of ma̅maki leaves and/or fruit and/or seeds with food in accordance with the desired number of times and frequency of intake, it is possible to provide the food product of the present invention that can be expected to have the desired effect according to the type of desired action and effect.

The food product of the present invention is characterized by containing the agent of the present invention, and is extremely useful since it shares various advantages of the agent of the present invention. In addition, as it is a food product, it can be taken safely and conveniently not only by patients suffering from specific diseases but also by healthy people.

In addition, the food product of the present invention can be used not only for humans but also for animals other than humans to which the agent of the present invention can be applied.

### [Pharmaceutical product]

One aspect of the present invention provides a pharmaceutical product containing the agent of the present invention (the pharmaceutical product of the present invention). The pharmaceutical product of the present invention is characterized by containing any one of the agents of the present invention and is used for the purpose of the agent.

The route of administration of the pharmaceutical product of the present invention is not limited, but it may usually be an oral medicine (oral agent) administered orally.

The pharmaceutical product of the present invention can be manufactured in any dosage form by adding the agent of the present invention as an active ingredient. Examples of such dosage forms include: a capsule preparation, such as a tablet, soft capsule, or hard capsule; a solid preparation, such as a powder, granule, drop, or pill; a semi-solid preparation, such as a jelly; and a liquid preparation, such as a syrup, suspension, or internal liquid. In this case, the pharmaceutical product of the present invention can be formulated as a pharmaceutical composition combining the agent of the present invention with other additives commonly used in the manufacture of oral agents, using pharmaceutical manufacturing methods known to those skilled in the art.

Examples of additives that can be used in the manufacture of the pharmaceutical product of the present invention include excipients, disintegrants, binders, lubricants, coating agents, dispersants, fluidizing agents, stabilizers, preservatives, buffering agents, masking agents, suspending agents, emulsifiers, fragrances, solubility aids, colorants, and thickening agents. The agent of the present invention may also be combined with a pharmaceutically acceptable carrier to provide the pharmaceutical product of the present invention with further enhanced action and effects.

The pharmaceutical product of the present invention may be a product that is deemed to be equivalent to a pharmaceutical under the legal system of each country. Examples include quasi-drugs in Japan.

The content of the active ingredients of the agent of the present invention, i.e., ma̅maki leaves and/or fruit and/or seeds, in the pharmaceutical product of the present invention can be set appropriately within the range in which the effect of the present invention can be obtained, as with the agent of the present invention. Specifically, the content of the active ingredients of the agent of the present invention, i.e., ma̅maki leaves and/or fruit and/or seeds, may be adjusted to within the range of usually 0.01 mg/day or more, or 0.1 mg/day or more, preferably 0.5 mg/day or more, more preferably 1.0 mg/day or more, and usually 10 g/day or less, preferably 5 g/day or less, more preferably 1 g/day or less.

The number of times and frequency of administration of the pharmaceutical product of the present invention are arbitrary and can be set as appropriate at any number of times and frequency, such as once to several times a day, every day, every other day, every two days, or once to seven times a week. By using the desired amount of ma̅maki leaves and/or fruit and/or seeds in accordance with the desired number of times and frequency of administration, it is possible to provide the pharmaceutical product of the present invention that can be expected to have the desired effect according to the type of desired action and effect.

The pharmaceutical product of the present invention is characterized by containing the agent of the present invention, and is extremely useful since it shares various advantages of the agent of the present invention.

In addition, the pharmaceutical product of the present invention can be used not only for humans but also for animals other than humans to which the agent of the present invention can be applied.

### [Method]

One aspect of the present invention provides a method for maintaining or improving a cerebral function, a method for promoting nerve cell repair or inducing neurogenesis, a method for removing a neurodegenerative disease-causing protein that accumulates in the brain, or a method for treating or preventing a neurodegenerative disease (the method of the present invention), the method comprising administering to a subject one or more selected from an herbal medicine selected from ma̅maki leaves, fruit, and seeds, the agent of the present invention, the food product of the present invention, and the pharmaceutical product of the present invention.

The method of the present invention can be carried out by administering ma̅maki leaves and/or fruit and/or seeds, which are the active ingredients of the agent of the present invention, to a subject in the form of the agent of the present invention, the food product of the present invention, and/or the pharmaceutical product of the present invention. The details of the method of the present invention are the same as those described above for the agent of the present invention, the food product of the present invention, and the pharmaceutical product of the present invention.

### EXAMPLES

Hereinafter, the present invention will be described in more detail with reference to specific Examples. However, these Examples are merely provided for the purpose of explanation, and the present invention is not limited to these Examples in any way.

### [Materials and methods]

### *Preparation of hot water extract and unextracted simple crushed powder of ma̅maki leaves and fruit

Dried ma̅maki tea leaves were purchased from Nakihalani Farm, LLC (Hawaii, USA). The ma̅maki leaf hot water extract was prepared at TechnoPro L&D Co., Ltd. (Tokyo, Japan). 250 g of ma̅maki tea leaves were added to 3 L of water and soaked for 1 hour. After boiling for 15 minutes with stirring, the mixture was left to stand for 24 hours. After removing the tea leaves, suction filtration was performed using a filter paper with a pore size of 8 µm. The filtrate was concentrated to 500 mL by heating with an evaporator at 40°C. The concentrated liquid was freeze-dried to obtain 66 g of extract powder. Unextracted simple crushed powders of ma̅maki leaves and fruit were prepared in our laboratory. Since a small amount of fruit (including seeds) was mixed in the commercially available ma̅maki tea leaf package, the tea leaves and fruit were manually separated and each was crushed into particles of 20 to 50 µm using a grinder (Fine Powder Mill FM-100, Labonect, Sakai, Japan). These particles were collected and designated as unextracted simple crushed powder.

### *Component analysis of ma̅maki

Ma̅maki leaves are known to contain three main polyphenols: catechin, chlorogenic acid, and rutin (Non-Patent Literature 10: Chun et al., Native Hawaiian medicines, First People's Productions Honolulu, (1994), 216-217). The ma̅maki samples prepared above were sent to the Japan Food Research Laboratories (Tokyo, Japan) to measure the content of these polyphenols.

For catechin, 0.4 g of the substance was suspended in 30 mL of a mixture of methanol and oxalic acid (8:2) and extracted by shaking for 10 minutes. After centrifugation, the supernatant was collected, and the pellet was subjected to methanol extraction two more times. The supernatants from the three extractions were combined to a total volume of 100 mL. The extract was diluted and separated by high-performance liquid chromatography (HPLC) using a reverse-phase InertSustain C18 column (GL Sciences, Tokyo, Japan) with a mobile phase consisting of a mixture of 0.1% acetic acid and acetonitrile (89:11). The eluted fractions were analyzed sequentially using electrospray ionization (ESI)-mass spectrometry (MS) with a Xevo TQ MS (Waters Corporation, Milford, Massachusetts).

For chlorogenic acid, 0.2 g of the substance was suspended in 80 mL of a mixture of methanol and 0.02 M perchloric acid (1:9) and extracted by shaking for 10 minutes. After centrifugation, the supernatant was collected, and the pellet was subjected to methanol extraction two more times. The supernatants from the three extractions were combined to a total volume of 250 mL. The extract was separated by HPLC using a reverse-phase CAPCELL PAKC 18 ACR column (Osaka Soda, Osaka, Japan) with a mobile phase consisting of a mixture of water, acetonitrile, and phosphoric acid (920:80:2). The absorbance of the eluted fractions at 325 nm was measured.

Finally, for rutin, 0.4 g of the substance was suspended in 60 mL of a mixture of methanol and 2.5% acetic acid (8:2) and extracted by shaking for 10 minutes. After centrifugation, the supernatant was collected, and the pellet was subjected to methanol extraction two more times. The supernatants from the three extractions were combined to a total volume of 200 mL. The extract was diluted and separated by HPLC using a reverse-phase Unison UK-C18 column (Imtakt USA, Portland, Oregon, USA) with a mobile phase consisting of a mixture of water containing 0.4% citric acid, acetonitrile, and 2-propanol (200:38:2). The absorbance of the eluted fractions at 360 nm was measured.

### *Mice

Four different neurodegenerative dementia mouse models were used.

The Tau784 mice are FTD models that express both three-repeat and four-repeat human tau at an adult age (with four-repeat human tau being dominantly expressed) due to the presence of a tau intron mutation (Non-Patent Literature 25: Umeda et al., Am. J. Pathol., (2013), 183[1]:211-25; and Non-Patent Literature 26: Umeda et al., Ann. Clin. Transl. Neurol., (2015), 2[3]:241-55). According to previous studies by the present inventors, due to the unbalanced expression of tau isoforms, these mice exhibit tau hyperphosphorylation, tau oligomer formation, synapse loss, and memory impairment at six months of age, microglia activation at twelve months of age, and neurofibrillary tangle formation and neuron loss at fifteen months of age.

The APP23 mice are AD models expressing human APP with the Swedish (KM670/671NL) mutation (Non-Patent Literature 27: Sturchler-Pierrat et al., Proc. Natl. Acad. Sci. U.S.A., (1997), 94[24]:13287-92; Non-Patent Literature 28: Van Dam et al., Eur. J. Neurosci., (2003), 17[2]:388-96; and Non-Patent Literature 29: Umeda et al., Front. Neurosci., (2021), 15:763476). These mice exhibit memory impairment at three months of age. According to previous studies by the present inventors, these mice exhibit accumulation of Aβ oligomers, synapse loss, and amyloid deposition at fifteen months of age.

The Huα-Syn (A53T) strain G2-3 mice were originally created as a PD model expressing human α-synuclein with the A53T mutation (Non-Patent Literature 30: Lee et al., Proc. Natl. Acad. Sci. U.S.A., (2002), 99[13]:8968-73; and Non-Patent Literature 31: Umeda et al., Int. J. Mol. Sci., (2021), 22:8453). According to previous studies by the present inventors, these mice exhibit accumulation of α-synuclein oligomers from four months after birth, cognitive impairment at six months after birth, and motor dysfunction at nine months after birth. Therefore, they can be considered a model of DLB until nine months after birth.

The C9-500 mice are FTD/ALS models into which the human full-length C9orf72 gene was introduced (Non-Patent Literature 32: Liu et al., Neuron, (2016), 90[3]:521-534; and Non-Patent Literature 33: Hatanaka et al., Biomedicines, (2022), 10[5]:1080). They have a mutation with an abnormal expansion (hexanucleotide repeat expansion, HRE) of the GGGGCC sequence in intron 1a of about 500 repeats and have been reported to exhibit various pathologies, including TDP-43 (Non-Patent Literature 32). We confirmed that these mice show accumulation of RNA G-quadruplex forming RNA foci, dipeptide repeat proteins (DPRs) such as poly-GA and poly-GP, and phosphorylated TDP-43 at three months of age, and synapse loss, neuronal cell loss, and microglia activation at six months of age (Non-Patent Literature 33). By the way, DPRs are produced by repeat-associated non-ATG (RAN) translation, which is regulated by double-stranded RNA-dependent protein kinase (PKR). The cognitive function of these mice begins to decline at 4.5 months of age, but their motor function remains normal until 12 months of age (Non-Patent Literature 33). Therefore, this mouse model can be considered an FTD-TDP model until 12 months of age. Transgenic mice (Tg) are crossed with wild-type FVB/N Jc1 mice to maintain heterozygous transgenes.

All transgenic (Tg) mice were maintained and used as heterozygous animals. All animal experiments were approved by the Ethics Committee of Osaka Metropolitan University (Osaka, Japan) and conducted in accordance with the Animal Experiment Guide of Osaka Metropolitan University.

### *Mouse treatment

To investigate the effect of ma̅maki leaf hot water extract, the extract powder was suspended in water at concentrations of 3.33, 0.33, and 0.10 mg/mL by sonication. 300 µL of each suspension (containing 1,000, 100, and 30 µg of powder) was orally administered to male and female Tau784 mice five days a week (Monday to Friday) for one month. As a control, the same amount of water was administered to age-matched Tg and non-Tg littermates. For APP23, Huα-Syn (A53T), and C9-500 mice, a 0.33 mg/mL suspension (100 µg of powder/300 µL) was orally administered to male and female mice for one month.

To compare the effects of the three ma̅maki preparations-ma̅maki leaf hot water extract, ma̅maki leaf simple crushed powder, and ma̅maki fruit simple crushed powder-each powder was suspended in water at a concentration of 0.10 mg/mL by sonication. 300 µL of each suspension (containing 30 µg of powder) was orally administered to Tau784 mice for one month.

To investigate the effects of the polyphenols contained in ma̅maki, catechin, chlorogenic acid, and rutin (all from Fujifilm-Wako, Osaka, Japan) were placed in one tube and dissolved in water to prepare a mixed solution. The concentrations were adjusted to 0.29, 0.12, and 0.41 µg/mL, respectively. 300 µL of the mixed solution (containing 0.087 µg of catechin, 0.036 µg of chlorogenic acid, and 0.123 µg of rutin) was administered to Tau784 mice for one month. The doses of these polyphenols correspond to the amount contained in 30 µg of the ma̅maki fruit crushed powder. A solution containing only catechin (0.087 µg/300 µL) was also administered to Tau784 mice.

### *Behavior tests

The spatial reference memory of each mouse was evaluated using the Morris water maze test (Non-Patent Literature 34: Kelley Bromley-Brits et al., J. Vis. Exp., (2011), 53e2920). A platform with a diameter of 10 cm was placed 1 cm below the water surface in a 1-meter-diameter circular pool so that it was invisible to the mice. Each mouse was placed in the pool and allowed to swim for 60 seconds, and the time taken to reach the platform was measured (referred to as escape latency). If the mouse did not reach the platform within 60 seconds, the experimenter placed the mouse on the platform and allowed it to rest for a short period. This trial was conducted five times per day at five-minute intervals for four consecutive days for each mouse. This test procedure was used to measure memory acquisition ability.

### *Histological analysis of neuropathology

Following the behavior tests, the mice from each group were divided into two subgroups, one for histological analysis and the other for subsequent biochemical analysis. For the mice for histological analysis, after perfusion-fixation with 4% paraformaldehyde, brain sections were prepared according to previously reported methods (Non-Patent Literature 27: Sturchler-Pierrat et al., Proc. Natl. Acad. Sci. U.S.A., (1997), 94[24]:13287-92; and Non-Patent Literature 33: Hatanaka et al., Biomedicines, (2022), 10[5]:1080) and neuropathology was stained by immunohistochemistry. The antibodies used for immunohistochemistry are shown in the table below. The pathology was evaluated by quantifying the staining intensity or staining area in a specific brain region using NIH Image J software. Neuroinflammation (Non-Patent Literature 35: Muzio et al., Front Neurosci., (2021), 24[15]:742065) was evaluated by counting the number of Iba-1 positive activated microglia in a specific brain region. The correspondence between each neuropathology and the detection antibody is shown in the table below.

**TABLE 1**

| Neuropathology | Antibody |
|---|---|
| Phosphorylated tau | AT8 antibody (recognizes pSer202/Thr205) (Thermo Scientific, Waltham, MA) |
| Tau oligomers | T22 antibody (Sigma-Aldrich, St Louis, MO) |
| Aβ oligomers | 11A1 antibody (IBL, Fujioka, Japan) |
| Amyloid deposition | β001 antibody (in-house) (Non-Patent Literature 27) |
| Phosphorylated α-synuclein | EP1536Y antibody (recognizes pSer129) (Abcam, Cambridge, UK) |
| α-Synuclein oligomers | Syn33 antibody (Sigma-Aldrich) |
| DNA/RNA G-quadruplex | BG4 antibody (Absolute antibody, Cleveland, UK) |
| poly-GA, poly-GP | Anti-poly-GA antibody, anti-poly-GP antibody (both from Cosmo Bio, Tokyo, Japan) |
| Phosphorylated TDP-43 | Anti-phospho-TDP-43 antibody (recognizes pSer409/410) (Cosmo Bio) |
| Phosphorylated PKR | Anti-phospho-PKR antibody (recognizes pThr446) (MilliporeSigma, Burlington, MA) |
| Synaptophysin (synapse marker) | SVP-38 antibody (Sigma-Aldrich) |
| Activated microglia | Anti-Iba-1 antibody (Fujifilm-Wako) |

### *Histological analysis of BDNF expression and neurogenesis

BDNF expression was evaluated in Tau784 mice administered the three ma̅maki preparations at 30 µg/day for one month. Brain sections were stained with an anti-BDNF antibody (GTX132621; GeneTex, Irvine, CA), and the staining intensity in a specific brain region was quantified using NIH Image J software.

Neurogenesis was evaluated using old Huα-Syn (A53T) mice. The mice were orally administered a ma̅maki fruit simple crushed powder suspension at 30 µg/day for one month. 5-bromo-2'-deoxyuridine (BrdU; Sigma-Aldrich), a thymidine analogue that is selectively incorporated into the DNA of proliferating cells, was dissolved at 5 mg/mL in Tris-buffered saline, pH 7.6, and 300 µL of this solution (containing 1.5 mg) was administered daily via intraperitoneal injection to the mice for the final five days of ma̅maki administration. Brain sections were double-stained with a mouse monoclonal anti-BrdU antibody (IBL) and a rabbit polyclonal anti-doublecortin antibody (Abcam). Cells positive for both of these two antibodies were regarded as newly born neurons, and their number was counted in a specific brain region.

### *Statistical analysis

For comparisons of averages between three or more groups, ANOVA or two-factor repeated measures ANOVA (for behavioral tests) was used, followed by Fisher's PLSD test. A p-value < 0.05 was considered to indicate a significant difference.

### [Example 1] Study on the effect of ma̅maki leaf hot water extract (Leaf-ext) on Tau784 mice

The effect of ma̅maki leaf hot water extract (Leaf-ext) on Tau784 mice was investigated. Tau784 mice aged 12-14 months (average body weight 31.8 g) were orally administered ma̅maki leaf hot water extract (Leaf-ext) powder at 1000 µg/day, 100 µg/day, or 30 µg/day for one month. As a control, the same amount of water was administered to age-matched Tau784 mice and non-genetically modified littermates.

Figure 1 is a graph showing the results of the Morris water maze test for Tau784 mice administered ma̅maki leaf hot water extract (Leaf-ext) orally for one month (labeled "Tg + Leaf-ext" in the table), in comparison with Tau784 mice administered water (labeled "Tg" in the table) and non-genetically modified mice (labeled "Non-Tg" in the table). Fig. 1A is a graph showing the results for the 1000 µg/day and 100 µg/day dose groups, and Fig. 1B is a graph showing the results for the 30 µg/day dose group. As is clear from these results, the memory of the Tau784 mice was improved in a dose-dependent manner by the administration of ma̅maki leaf hot water extract (Leaf-ext). In particular, in the groups with doses of 1000 µg/day and 100 µg/day, the memory of the Tau784 mice was improved to a level equivalent to that of non-genetically modified littermates.

Figure 2 shows tau pathology in the entorhinal cortex of Tau784 mice administered ma̅maki leaf hot water extract (Leaf-ext) at a dose of 100 µg/day orally for one month (labeled "Tau784 + Leaf-ext" in the table), in comparison with Tau784 mice administered water (labeled "Tau784" in the table). Fig. 2A shows photographs of the staining results for phosphorylated tau and tau oligomers, and Fig. 2B shows graphs of the quantitative values of the staining intensity in each photograph. As is clear from these results, the levels of phosphorylated tau and tau oligomers in the Tau784 mice were significantly reduced by the administration of ma̅maki leaf hot water extract (Leaf-ext).

Figure 3 shows synaptophysin pathology in the hippocampal CA2/3 region of Tau784 mice administered ma̅maki leaf hot water extract (Leaf-ext) at a dose of 100 µg/day orally for one month (labeled "Tau784 + Leaf-ext" in the table), in comparison with Tau784 mice administered water (labeled "Tau784" in the table) and non-genetically modified mice (labeled "Non-Tg" in the table). Fig. 3A shows photographs of the staining results for synaptophysin, and Fig. 3B shows graphs of the quantitative values of the staining intensity in each photograph. As is clear from these results, the synaptophysin level in the hippocampal CA2/3 region of the Tau784 mice was improved to a level equivalent to that of non-genetically modified littermates by the administration of ma̅maki leaf hot water extract (Leaf-ext).

Figure 4 shows microglia pathology in the hippocampus (HC) and cerebral cortex (CTX) of Tau784 mice administered ma̅maki leaf hot water extract (Leaf-ext) at a dose of 100 µg/day orally for one month (labeled "Tau784 + Leaf-ext" in the table), in comparison with Tau784 mice administered water (labeled "Tau784" in the table) and non-genetically modified mice (labeled "Non-Tg" in the table). Fig. 4A shows photographs of the staining results for activated microglia, and Fig. 4B shows graphs of the number of positive cells in each photograph. As is clear from these results, the activated microglia levels in both the hippocampus (HC) and cerebral cortex (CTX) of the Tau784 mice were improved to a level equivalent to that of non-genetically modified littermates by the administration of ma̅maki leaf hot water extract (Leaf-ext).

### [Example 2] Study on the effect of ma̅maki leaf hot water extract (Leaf-ext) on APP23 mice

The effect of ma̅maki leaf hot water extract (Leaf-ext) on APP23 mice was investigated. APP23 mice aged 14-17 months (average body weight 30.1 g) were orally administered ma̅maki leaf hot water extract (Leaf-ext) powder at 100 µg/day for one month. As a control, the same amount of water was administered to age-matched APP23 mice and non-genetically modified littermates.

Figure 5 is a graph showing the results of the Morris water maze test for APP23 mice administered ma̅maki leaf hot water extract (Leaf-ext) at a dose of 100 µg/day orally for one month (labeled "Tg + Leaf-ext" in the table), in comparison with APP23 mice administered water (labeled "Tg" in the table) and non-genetically modified mice (labeled "Non-Tg" in the table). As is clear from these results, the memory of the APP23 mice was improved to a level close to that of non-genetically modified littermates (non-Tg) by the administration of ma̅maki leaf hot water extract (Leaf-ext).

Figure 6 shows amyloid pathology in the cerebral cortex and hippocampus of APP23 mice administered ma̅maki leaf hot water extract (Leaf-ext) at a dose of 100 µg/day orally for one month (labeled "APP23 + Leaf-ext" in the table), in comparison with APP23 mice administered water (labeled "APP23" in the table). Fig. 6A shows photographs of the staining results for amyloid deposition and Aβ oligomers, and Fig. 6B shows graphs of the quantitative values of the staining intensity in each photograph. As is clear from these results, the levels of amyloid deposition and Aβ oligomers in the APP23 mice were significantly reduced by the administration of ma̅maki leaf hot water extract (Leaf-ext).

Figure 7 shows synaptophysin pathology in the hippocampal CA2/3 region of APP23 mice administered ma̅maki leaf hot water extract (Leaf-ext) at a dose of 100 µg/day orally for one month (labeled "APP23 + Leaf-ext" in the table), in comparison with APP23 mice administered water (labeled "APP23" in the table) and non-genetically modified mice (labeled "Non-Tg" in the table). Fig. 7A shows photographs of the staining results for synaptophysin, and Fig. 7B shows graphs of the quantitative values of the staining intensity in each photograph. As is clear from these results, the synaptophysin level in the hippocampal CA2/3 region of the APP23 mice was improved to a level equivalent to that of non-genetically modified littermates by the administration of ma̅maki leaf hot water extract (Leaf-ext).

Figure 8 shows microglia pathology in the hippocampus (HC) and cerebral cortex (CTX) of APP23 mice administered ma̅maki leaf hot water extract (Leaf-ext) at a dose of 100 µg/day orally for one month (labeled "APP23 + Leaf-ext" in the table), in comparison with APP23 mice administered water (labeled "APP23" in the table) and non-genetically modified mice (labeled "Non-Tg" in the table). Fig. 8A shows photographs of the staining results for activated microglia, and Fig. 8B shows graphs of the number of positive cells in each photograph. As is clear from these results, the activated microglia levels in both the hippocampus (HC) and cerebral cortex (CTX) of the APP23 mice were improved to a level close to that of non-genetically modified littermates by the administration of ma̅maki leaf hot water extract (Leaf-ext).

### [Example 3] Study on the effect of ma̅maki leaf hot water extract (Leaf-ext) on Huα-Syn (A53T) mice

The effect of ma̅maki leaf hot water extract (Leaf-ext) on Huα-Syn (A53T) mice was investigated. Huα-Syn (A53T) mice aged 7-8 months (average body weight 28.4 g) were orally administered ma̅maki leaf hot water extract (Leaf-ext) powder at 100 µg/day for one month. As a control, the same amount of water was administered to age-matched Huα-Syn (A53T) mice and non-genetically modified littermates.

Figure 9 is a graph showing the results of the Morris water maze test for Huα-Syn (A53T) mice administered ma̅maki leaf hot water extract (Leaf-ext) at a dose of 100 µg/day orally for one month (labeled "Tg + Leaf-ext" in the table), in comparison with Huα-Syn (A53T) mice administered water (labeled "Tg" in the table) and non-genetically modified mice (labeled "Non-Tg" in the table). As is clear from these results, the memory of the Huα-Syn (A53T) mice was improved to a level close to that of non-genetically modified littermates (non-Tg) by the administration of ma̅maki leaf hot water extract (Leaf-ext).

Figs. 10 and 11 show α-synuclein pathology in the hippocampus (HC) and entorhinal cortex (EC) of Huα-Syn (A53T) mice administered ma̅maki leaf hot water extract (Leaf-ext) at a dose of 100 µg/day orally for one month (labeled "αSyn-Tg + Leaf-ext" in the table), in comparison with Huα-Syn (A53T) mice administered water (labeled "αSyn-Tg" in the table). Fig. 10A shows photographs of the staining results for phosphorylated α-synuclein, Fig. 11A shows photographs of the staining results for α-synuclein oligomers, and Figs. 10B and 11B show graphs of the quantitative values of the staining intensity in each photograph. As is clear from these results, the levels of phosphorylated α-synuclein and α-synuclein oligomers in the Huα-Syn (A53T) mice were significantly reduced by the administration of ma̅maki leaf hot water extract (Leaf-ext).

Figure 12 shows synaptophysin pathology in the hippocampal CA2/3 region of Huα-Syn (A53T) mice administered ma̅maki leaf hot water extract (Leaf-ext) at a dose of 100 µg/day orally for one month (labeled "αSyn-Tg + Leaf-ext" in the table), in comparison with Huα-Syn (A53T) mice administered water (labeled "αSyn-Tg" in the table) and non-genetically modified mice (labeled "Non-Tg" in the table). Fig. 12A shows photographs of the staining results for synaptophysin, and Fig. 12B shows graphs of the quantitative values of the staining intensity in each photograph. As is clear from these results, the synaptophysin level in the hippocampal CA2/3 region of the Huα-Syn (A53T) mice was improved to a level equivalent to that of non-genetically modified littermates by the administration of ma̅maki leaf hot water extract (Leaf-ext).

Figure 13 shows microglia pathology in the hippocampus (HC) and cerebral cortex (CTX) of Huα-Syn (A53T) mice administered ma̅maki leaf hot water extract (Leaf-ext) at a dose of 100 µg/day orally for one month (labeled "αSyn-Tg + Leaf-ext" in the table), in comparison with Huα-Syn (A53T) mice administered water (labeled "αSyn-Tg" in the table) and non-genetically modified mice (labeled "Non-Tg" in the table). Fig. 13A shows photographs of the staining results for activated microglia, and Fig. 13B shows graphs of the number of positive cells in each photograph. As is clear from these results, the activated microglia levels in both the hippocampus (HC) and cerebral cortex (CTX) of the Huα-Syn (A53T) mice were improved to a level close to that of non-genetically modified littermates by the administration of ma̅maki leaf hot water extract (Leaf-ext).

### [Example 4] Study on the effect of ma̅maki leaf hot water extract (Leaf-ext) on C9-500 mice

The effect of ma̅maki leaf hot water extract (Leaf-ext) on C9-500 mice was investigated. C9-500 mice aged 9-10 months (average body weight 29.2 g) were orally administered ma̅maki leaf hot water extract (Leaf-ext) powder at 100 µg/day for one month. As a control, the same amount of water was administered to age-matched C9-500 mice and non-genetically modified littermates.

Figure 14 is a graph showing the results of the Morris water maze test for C9-500 mice administered ma̅maki leaf hot water extract (Leaf-ext) at a dose of 100 µg/day orally for one month (labeled "Tg + Leaf-ext" in the table), in comparison with C9-500 mice administered water (labeled "Tg" in the table) and non-genetically modified mice (labeled "Non-Tg" in the table). As is clear from these results, the memory of the C9-500 mice was improved to a level close to that of non-genetically modified littermates (non-Tg) by the administration of ma̅maki leaf hot water extract (Leaf-ext).

Figure 15 shows pathology resulting from a mutation in the C9orf72 gene in the prefrontal cortex (PFC) of C9-500 mice administered ma̅maki leaf hot water extract (Leaf-ext) at a dose of 100 µg/day orally for one month (labeled "C9-500 + Leaf-ext" in the table), in comparison with C9-500 mice administered water (labeled "C9-500" in the table). Fig. 15A shows photographs of the staining results for RNA G-quadruplex, poly-GA, poly-GP, and phosphorylated TDP-43, and Fig. 15B shows graphs of the quantitative values of the staining intensity in each photograph. As is clear from these results, the levels of pathology resulting from a mutation in the C9orf72 gene in the prefrontal cortex (PFC) of the C9-500 mice were significantly reduced by the administration of ma̅maki leaf hot water extract (Leaf-ext).

Figure 16 shows double-stranded RNA-dependent protein kinase (PKR) pathology in the prefrontal cortex (PFC) of C9-500 mice administered ma̅maki leaf hot water extract (Leaf-ext) at a dose of 100 µg/day orally for one month (labeled "C9-500 + Leaf-ext" in the table), in comparison with C9-500 mice administered water (labeled "C9-500" in the table) and non-genetically modified mice (labeled "Non-Tg" in the table). Fig. 16A shows photographs of the staining results for phosphorylated PKR, and Fig. 16B shows graphs of the quantitative values of the staining intensity in each photograph. As is clear from these results, the PKR levels in the prefrontal cortex (PFC) of the C9-500 mice were reduced to a level equivalent to that of non-genetically modified littermates by the administration of ma̅maki leaf hot water extract (Leaf-ext).

Figure 17 shows synaptophysin pathology in the hippocampal CA2/3 region of C9-500 mice administered ma̅maki leaf hot water extract (Leaf-ext) at a dose of 100 µg/day orally for one month (labeled "C9-500 + Leaf-ext" in the table), in comparison with C9-500 mice administered water (labeled "C9-500" in the table) and non-genetically modified mice (labeled "Non-Tg" in the table). Fig. 17A shows photographs of the staining results for synaptophysin, and Fig. 17B shows graphs of the quantitative values of the staining intensity in each photograph. As is clear from these results, the synaptophysin level in the hippocampal CA2/3 region of the C9-500 mice was improved to a level equivalent to that of non-genetically modified littermates by the administration of ma̅maki leaf hot water extract (Leaf-ext).

Figure 18 shows microglia pathology in the prefrontal cortex (PFC) of C9-500 mice administered ma̅maki leaf hot water extract (Leaf-ext) at a dose of 100 µg/day orally for one month (labeled "C9-500 + Leaf-ext" in the table), in comparison with C9-500 mice administered water (labeled "C9-500" in the table) and non-genetically modified mice (labeled "Non-Tg" in the table). Fig. 18A shows photographs of the staining results for activated microglia, and Fig. 18B shows graphs of the number of positive cells in each photograph. As is clear from these results, the activated microglia levels in the prefrontal cortex (PFC) of the C9-500 mice were improved to a level close to that of non-genetically modified littermates by the administration of ma̅maki leaf hot water extract (Leaf-ext).

### [Example 5] Study on the effect of ma̅maki leaf hot water extract (Leaf-ext), leaf simple crushed powder (Leaf-pwd), and fruit (including seeds) simple crushed powder (Fruit-pwd) on Tau784 mice

The effects of ma̅maki leaf hot water extract (Leaf-ext), ma̅maki leaf simple crushed powder (Leaf-pwd), and ma̅maki fruit (including seeds) simple crushed powder (Fruit-pwd) on Tau784 mice were investigated. Tau784 mice aged 7-9 months (average body weight 28.9 g) were orally administered ma̅maki leaf hot water extract (Leaf-ext) powder, leaf simple crushed powder (Leaf-pwd), and fruit (including seeds) simple crushed powder (Fruit-pwd) at 30 µg/day for one month. As a control, the same amount of water was administered to age-matched Tau784 mice and non-genetically modified littermates.

Figure 19 is a graph showing the results of the Morris water maze test for Tau784 mice administered ma̅maki leaf hot water extract (Leaf-ext), ma̅maki leaf simple crushed powder (Leaf-pwd), and ma̅maki fruit (including seeds) simple crushed powder (Fruit-pwd) at a dose of 30 µg/day orally for one month (labeled "Tg + Leaf-ext," "Tg + Leaf-pwd," and "Tg + Fruit-pwd" respectively in the table), in comparison with Tau784 mice administered water (labeled "Tg" in the table) and non-genetically modified mice (labeled "Non-Tg" in the table). As is clear from these results, the memory of the Tau784 mice was improved by the administration of ma̅maki leaf hot water extract (Leaf-ext), but it was not as good as that of the non-genetically modified littermates. With the administration of ma̅maki leaf simple crushed powder (Leaf-pwd), the memory of the Tau784 mice was improved to a level equivalent to that of the non-genetically modified littermates. With the administration of ma̅maki fruit (including seeds) simple crushed powder (Fruit-pwd), surprisingly, the memory of the Tau784 mice was significantly enhanced to a level even higher than that of the non-genetically modified littermates.

Figure 20 shows tau pathology in the entorhinal cortex of Tau784 mice administered ma̅maki leaf hot water extract (Leaf-ext), ma̅maki leaf simple crushed powder (Leaf-pwd), and ma̅maki fruit (including seeds) simple crushed powder (Fruit-pwd) at a dose of 30 µg/day orally for one month (labeled "Tg + Leaf-ext," "Tg + Leaf-pwd," and "Tg + Fruit-pwd" respectively in the table), in comparison with Tau784 mice administered water (labeled "Tau784" in the table). Fig. 20A shows photographs of the staining results for phosphorylated tau and tau oligomers, and Fig. 20B shows graphs of the quantitative values of the staining intensity in each photograph. As is clear from these results, the level of phosphorylated tau in the entorhinal cortex (EC) was significantly reduced by the administration of ma̅maki leaf simple crushed powder (Leaf-pwd) and ma̅maki fruit (including seeds) simple crushed powder (Fruit-pwd), but the effect was stronger with the fruit (including seeds) simple crushed powder (Fruit-pwd). On the other hand, the leaf hot water extract (Leaf-ext) showed only a slight effect. Meanwhile, the level of tau oligomers was significantly reduced by all administrations, with the effect being highest for the fruit (including seeds) simple crushed powder (Fruit-pwd) and weakest for the leaf hot water extract (Leaf-ext).

Figure 21 shows synaptophysin pathology in the hippocampal CA2/3 region of Tau784 mice administered ma̅maki leaf hot water extract (Leaf-ext), ma̅maki leaf simple crushed powder (Leaf-pwd), and ma̅maki fruit (including seeds) simple crushed powder (Fruit-pwd) at a dose of 30 µg/day orally for one month (labeled "Tg + Leaf-ext," "Tg + Leaf-pwd," and "Tg + Fruit-pwd" respectively in the table), in comparison with Tau784 mice administered water (labeled "Tau784" in the table) and non-genetically modified mice (labeled "Non-Tg" in the table). Fig. 21A shows photographs of the staining results for synaptophysin, and Fig. 21B shows graphs of the quantitative values of the staining intensity in each photograph. As is clear from these results, the synaptophysin level in the hippocampal CA2/3 region of the Tau784 mice was significantly recovered by the administration of ma̅maki leaf simple crushed powder (Leaf-pwd) and ma̅maki fruit (including seeds) simple crushed powder (Fruit-pwd), but the effect was stronger with the fruit (including seeds) simple crushed powder (Fruit-pwd). On the other hand, it was only incompletely recovered with the leaf hot water extract (Leaf-ext).

Figure 22 shows BDNF expression in the cerebral cortex (CTX) of Tau784 mice administered ma̅maki leaf hot water extract (Leaf-ext), ma̅maki leaf simple crushed powder (Leaf-pwd), and ma̅maki fruit (including seeds) simple crushed powder (Fruit-pwd) at a dose of 30 µg/day orally for one month (labeled "Tg + Leaf-ext," "Tg + Leaf-pwd," and "Tg + Fruit-pwd" respectively in the table), in comparison with Tau784 mice administered water (labeled "Tau784" in the table) and non-genetically modified mice (labeled "Non-Tg" in the table). Fig. 22A shows photographs of the staining results for BDNF, and Fig. 22B shows graphs of the quantitative values of the staining intensity in each photograph. As is clear from these results, the BDNF level in the cerebral cortex (CTX) of the Tau784 mice was significantly increased to a level higher than that of the non-genetically modified littermates with the ma̅maki fruit (including seeds) simple crushed powder (Fruit-pwd), and to a level equivalent to that of the non-genetically modified littermates with the leaf simple crushed powder (Leaf-pwd), while the leaf hot water extract (Leaf-ext) showed only a slight effect.

### [Example 6] Study on the effect of ma̅maki fruit (including seeds) simple crushed powder (Fruit-pwd) on Huα-Syn (A53T) mice

The effect of ma̅maki fruit (including seeds) simple crushed powder (Fruit-pwd) on Huα-Syn (A53T) mice was investigated. Huα-Syn (A53T) mice aged 10-11 months (average body weight 28.9 g) were orally administered ma̅maki fruit (including seeds) simple crushed powder (Fruit-pwd) at 30 µg/day for one month. As a control, the same amount of water was administered to age-matched Huα-Syn (A53T) mice and non-genetically modified littermates.

Figure 23 shows neurogenesis levels in the dentate gyrus (DG) and substantia nigra (SN) of Huα-Syn (A53T) mice administered ma̅maki fruit (including seeds) simple crushed powder (Fruit-pwd) at a dose of 30 µg/day orally for one month (labeled "αSyn-Tg + Fruit-pwd" in the table), in comparison with Huα-Syn (A53T) mice administered water (labeled "αSyn-Tg" in the table) and non-genetically modified mice (labeled "Non-Tg" in the table). Fig. 23A shows immunofluorescence staining photographs for BrdU (red) and doublecortin (DCX) (green). Double-positive cells (yellow), which are positive for both BrdU (red) and DCX (green), were regarded as newborn neurons. Fig. 23B shows graphs of the quantitative results of counting the number of double-positive cells (yellow) in each photograph. As is clear from these results, the neurogenesis levels in the dentate gyrus (DG) and substantia nigra (SN) of the Huα-Syn (A53T) mice were, surprisingly, improved to a level far exceeding that of the non-genetically modified littermates by the administration of ma̅maki fruit (including seeds) simple crushed powder (Fruit-pwd). Such results suggest that ma̅maki fruit (including seeds) has the effect of promoting brain rejuvenation through neuronal repair and regeneration.

### [Example 7] Study on the effect of the three polyphenols on Tau784 mice

The three polyphenols contained in ma̅maki, namely catechin, chlorogenic acid, and rutin, have all been reported to have anti-dementia effects. Therefore, the various effects of ma̅maki leaf hot water extract (Leaf-ext), leaf simple crushed powder (Leaf-pwd), and fruit (including seeds) simple crushed powder (Fruit-pwd) could be attributed to these polyphenols. Thus, the effects of the three polyphenols-catechin, chlorogenic acid, and rutin-on Tau784 mice were investigated.

First, the content of the three polyphenols-catechin, chlorogenic acid, and rutin-in 100 g of each of the ma̅maki leaf hot water extract (Leaf-ext), ma̅maki leaf simple crushed powder (Leaf-pwd), and ma̅maki fruit (including seeds) simple crushed powder (Fruit-pwd) was quantified. The results are shown in the table below.

**TABLE 2**

| Content of three polyphenols in 100 g of ma̅maki preparations | | | |
|---|---|---|---|
| Polyphenol | Leaf-ext | Leaf-pwd | Fruit-pwd |
| Catechin | 0.031 g | 0.15 g | 0.29 g |
| Chlorogenic acid | 0.44 g | 0.72 g | 0.12 g |
| Rutin | 0.83 g | 1.3 g | 0.41 g |

Next, Tau784 mice aged 8-10 months (average body weight 32.0 g) were orally administered a mixture of catechin at 0.087 µg, chlorogenic acid at 0.036 µg, and rutin at 0.123 µg, or catechin alone at 0.087 µg, for one month. The doses of these polyphenols corresponded to the amounts contained in 30 µg of ma̅maki fruit (including seeds) simple crushed powder. As a control, the same amount of water was administered to age-matched Huα-Syn (A53T) mice and non-genetically modified littermates.

Figure 24 is a graph showing the results of the Morris water maze test for Tau784 mice administered a mixture of catechin at 0.087 µg, chlorogenic acid at 0.036 µg, and rutin at 0.123 µg (labeled "Tg + 3 polyphenol mixture" in the table), or catechin only at 0.087 µg (labeled "Tg + catechin" in the table), orally for one month, in comparison with Tau784 mice administered water (labeled "Tg" in the table) and non-genetically modified mice (labeled "Non-Tg" in the table). As is clear from these results, the administration of the mixture of the three polyphenols improved the memory of the Tau784 mice, but the effect was far inferior to that of the non-genetically modified littermates and was incomplete. Furthermore, the administration of catechin alone showed a weaker effect than the polyphenol mixture. A comparison of these results with the effects in Examples 1 to 6 shows that the various effects of ma̅maki leaf hot water extract (Leaf-ext), leaf simple crushed powder (Leaf-pwd), and fruit (including seeds) simple crushed powder (Fruit-pwd) are not caused solely by the three polyphenols, but rather that other unknown components contained in ma̅maki contribute significantly.

### INDUSTRIAL APPLICABILITY

The present invention can be widely applied in fields such as functional foods and pharmaceuticals where maintenance or improvement of cognitive function is required, and therefore has an extremely high utility value.

## Claims

1. An agent for maintaining or improving a cerebral function, comprising an herbal medicine selected from ma̅maki leaves, fruit, and seeds.

2. The agent for maintaining or improving a cerebral function according to claim 1, wherein the cerebral function is cognitive function.

3. An agent for promoting nerve cell repair or inducing neurogenesis, comprising an herbal medicine selected from ma̅maki leaves, fruit, and seeds.

4. An agent for removing a neurodegenerative disease-causing protein that accumulates in the brain, comprising an herbal medicine selected from ma̅maki leaves, fruit, and seeds.

5. The agent according to claim 4, wherein the neurodegenerative disease-causing protein is one or more proteins selected from amyloid β (Aβ), tau, α-synuclein, TDP-43, FUS/TLS, polyglutamine, a protein resulting from RAN (repeat-associated non-ATG) translation, prion, and SOD-1.

6. An agent for treating or preventing a neurodegenerative disease, comprising an herbal medicine selected from ma̅maki leaves, fruit, and seeds.

7. The agent according to claim 6, wherein the neurodegenerative disease is degenerative dementia.

8. The agent according to claim 7, wherein the degenerative dementia is one or more dementias selected from Alzheimer's disease, frontotemporal dementia, Lewy body dementia, and dementia resulting from Parkinson's disease or amyotrophic lateral sclerosis.

9. The agent according to any one of claims 1 to 8, wherein the herbal medicine selected from ma̅maki leaves, fruit, and seeds is a crushed product and/or an extract of ma̅maki leaves and/or fruit and/or seeds.

10. The agent according to any one of claims 1 to 9, wherein the herbal medicine is administered to a subject at an amount of from 0.01 mg to 10 g per day.

11. A food product comprising the agent according to any one of claims 1 to 10.

12. A pharmaceutical product comprising the agent according to any one of claims 1 to 10.

13. A method for maintaining or improving a cerebral function in a subject, the method comprising administering to a subject in need thereof an herbal medicine selected from ma̅maki leaves, fruit, and seeds.

14. The method according to claim 13, wherein the cerebral function is cognitive function.

15. A method for promoting nerve cell repair or inducing neurogenesis in a subject, the method comprising administering to a subject in need thereof an herbal medicine selected from ma̅maki leaves, fruit, and seeds.

16. A method for removing a neurodegenerative disease-causing protein that accumulates in the brain of a subject, the method comprising administering to a subject in need thereof an herbal medicine selected from ma̅maki leaves, fruit, and seeds.

17. The method according to claim 16, wherein the neurodegenerative disease-causing protein is one or more proteins selected from amyloid β (Aβ), tau, α-synuclein, TDP-43, FUS/TLS, polyglutamine, a protein resulting from RAN (repeat-associated non-ATG) translation, prion, and SOD-1.

18. A method for treating or preventing a neurodegenerative disease in a subject, the method comprising administering to a subject in need thereof an herbal medicine selected from ma̅maki leaves, fruit, and seeds.

19. The method according to claim 18, wherein the neurodegenerative disease is degenerative dementia.

20. The method according to claim 19, wherein the degenerative dementia is one or more dementias selected from Alzheimer's disease, frontotemporal dementia, Lewy body dementia, and dementia resulting from Parkinson's disease or amyotrophic lateral sclerosis.

21. The method according to any one of claims 13 to 20, wherein the herbal medicine selected from ma̅maki leaves, fruit, and seeds is a crushed product and/or an extract of ma̅maki leaves and/or fruit and/or seeds.

22. The method according to any one of claims 13 to 21, wherein the herbal medicine is administered to a subject at an amount of from 0.01 mg to 10 g per day.
